# EUROPEAN PATENT SPECIFICATION

(11) **EP 1 011 795 B1**
(45) Date of publication and mention of the grant of the patent: **10.03.2010**
(21) Application number: 97929480.8
(22) Date of filing: 09.07.1997
(51) Int. Cl.: A61N 1/362, A61N 1/365

(54) **CARDIAC OUTPUT CONTROLLER**
GERÄT ZUR REGELUNG DES HERZZEITVOLUMENS
CONTROLEUR DU DEBIT CARDIAQUE

(30) Priority: 16.09.1996 US 26392 P; 17.09.1996 IL 11926196
(43) Date of publication of application: 28.06.2000
(73) Proprietor: IMPULSE DYNAMICS N.V., Curacao (AN)
(72) Inventor: BEN-HAIM, Shlomo, 34454 Haifa (IL); DARVISH, Nissim, 34606 Haifa (IL); MIKA, Yuval, 35567 Haifa (IL); FENSTER, Maier, 49600 Petach Tikva (IL)
(74) Representative: Fichter, Robert Arno
(86) International application number: PCT/IL1997/000235
(87) International publication number: WO 1998/010831

(56) References cited:
- EP-A- 0 727 241
- US-A- 5 083 564

## Description

### FIELD OF THE INVENTION

The present invention relates generally to cardiac therapeutic devices, and specifically to invasive devices for enhancing performance of the heart.

### BACKGROUND OF THE INVENTION

Cardiac insufficiency, characterized *inter alia* by a reduction in the cardiac output, is a common, well-known and well-documented heart malfunction. It develops as a result of congenital defects or as an end-effect of many diseases. Cardiac output, i.e., the output of the heart per unit time, is the product of stroke volume and heart rate. Hence, variations in cardiac output can be produced by changes in cardiac rate or stroke volume. The stroke volume can be influenced, for example, by changing the strength of cardiac contraction, by changing the length of the cardiac muscle fibers, and by changing contractility of cardiac muscle independent of fiber length. The heart rate and rhythm influence the cardiac output both directly and indirectly, since changes in the rate and rhythm also affect myocardial contractility.

The human body normally regulates the cardiac output in response to body needs by changing the heart rate, as during physical exercise, and/or by adapting the stroke volume. Under pathological conditions, however, some of the normal regulatory mechanisms may be damaged. For example, heart tissue damaged due to myocardial infarct typically cannot sustain normal pumping function, leading to a reduction in stroke volume, and hence of cardiac output. The body may react to such a reduction by increasing the heart rate, thus imposing long term strain on the heart muscles, leading in more severe cases to heart failure. There is thus a need for devices and treatments that can regulate the cardiac output, so as to compensate for the deficiencies in the normal regulation mechanisms.

In response to this need, modern cardiology has developed means to control various parameters associated with the heart's operation. Pharmaceuticals, for example, may be used to influence the conduction velocity, excitability, contractility and duration of the refractory period of the heart tissue. These pharmaceuticals are used to treat arrhythmia, enhance cardiac output and prevent fibrillation. Pharmaceuticals are generally limited in effectiveness in that they affect both healthy and diseased segments of the heart, usually, with a relatively low precision. They frequently also have unwanted side-effects.

A special kind of control can be achieved using implantable electronic devices, which provide excitatory electrical stimulation to the heart to control directly the heart rate and/or rhythm. For example, a pacemaker, an electronic devices which is typically implanted in the heart to support the heart's electrical excitation system or to bypass a blocked portion of the conduction system. Another type of cardiac electronic device is a defibrillator, which senses fibrillation in the heart and applies a high voltage impulse to "reset" the heart. While electronic pacemakers can control the heart rate, however, they are limited in their capacity to enhance cardiac output, and they are known to reduce stroke volume in at least some instances. Defibrillators are useful in treating arrhythmia when it occurs (although they are painful to the patient and traumatic to the heart), but they provide no long-term amelioration of cardiac insufficiency. In US 5,083,564 pacing stimuli and subthreshold stimuli are delivered for alleviating or diagnosing symptoms of heart block.

Thus, none of the treatments known in the art allow effective, long-term regulation of cardiac output. The electromechanical properties of the heart, as well as methods known in the art for influencing these properties, are more fully described in the "Background of the Invention" section of WO 9 725 098.

### SUMMARY OF THE INVENTION

It is an object of the present invention to provide a device for regulation of hemodynamic parameters, and particularly for increasing the cardiac output by enhancing the heart's stroke volume.

In preferred embodiments of the present invention, a cardiac output controller comprises a non-excitatory stimulation probe, including one or more non-excitatory stimulation electrodes; at least one sensor, preferably a sensing electrode; and electronic control circuitry, coupled to the stimulation probe and sensor. The stimulation electrodes and, preferably, the sensor are implanted in the heart of a subject. Alternatively, a sensing electrode may be placed on a body surface The circuitry receives signals from the sensor, indicative of the heart's activity, and responsive thereto, drives the stimulation electrodes to provide non-excitatory electrical stimulation to the heart.

The term "non-excitatory electrical stimulation," in the context of the present patent application and in the claims, refers to electrical pulses that do not induce new activation potentials to propagate in cardiac muscle cells. Rather, such pulses generally affect the response of the heart muscle to the action potentials, possibly by modulating cell contractility within selected segments of the cardiac muscle. Specifically, as described in the above-mentioned WO 9 725 098, the inventors have found that by applying non-excitatory electrical stimulation pulses of suitable strength, appropriately timed with respect to the heart's electrical activation, the contraction of the selected segments can be increased or decreased, thus increasing or decreasing the stroke volume of the heart. This finding forms the basis for the present invention.

In the present invention, characteristics of the non-excitatory stimulation are adjusted so as to modify the heart's muscular activity, thus affecting the cardiac output, preferably by increasing the stroke volume, without directly affecting the heart rate. Preferably, the device is used to increase cardiac output substantially continuously for extended periods of time, most preferably to boost cardiac output during those portions of a day for which a patient needs increased blood supply. Accordingly, the device is preferably not used at night, to allow the heart muscle to rest.

Alternatively, the device may be used to modify the heart's muscular activity in other ways. For example, in some conditions, such as HOCM (hypertrophic obstructive cardiomyopathy), the device may be operated to reduce cardiac output, so as to reduce the workload on the heart, or particularly to reduce cardiac muscle contraction in a hypertrophic region of the heart. As another example, the device may be used to increase the heart's contraction efficiency, so that a given level of cardiac output is maintained at a reduced expenditure of energy. Such uses of the device are described further in a PCT patent publication WO 9 810 828, entitled, "Apparatus and Method for Controlling the Contractility of Muscles."

In any case, the effect of the device on cardiac output is regulated by changing the timing of the non-excitatory stimulation pulse relative to the heart's activity, preferably relative to the heart's local electrical activity or ECG signals received by the sensing electrode, and/or by changing other pulse characteristics, such as the voltage, current, duration, polarity, waveform and frequency of the waveform. Preferably, the device senses the heart's sinus rhythm and applies and synchronizes the stimulation pulse relative thereto, preferably with a delay before the onset of the stimulation pulse. Additionally, the circuitry may analyze the signals, for example, to determine the QT interval, so as to adjust the stimulation pulses responsive thereto. Alternatively, when the heart's rhythm is irregular, due to ventricular premature beats (VPB's) or other cardiac arrhythmias, the device preferably identifies and analyzes the irregularity, using signal processing methods known in the art, and adjusts or withholds the stimulation pulse accordingly.

In some preferred embodiments of the present invention the control circuitry is contained within a console external to the body, and the electrodes are fed percutaneously into the subject's vascular system, for example, through the femoral artery, and are implanted in the heart. Such embodiments are useful particularly in short-term therapy to regulate and stabilize the subject's hemodynamics following an insult or trauma, for example, open heart surgery or MI.

In alternative preferred embodiments of the present invention, the electronic control circuitry is contained within a miniaturized, implantable case, similar to pacemaker cases known in the art.

In some preferred embodiments of the present invention, the non-excitatory stimulation electrodes have a large surface area in contact with the heart tissue, by comparison with intracardiac electrodes known in the art, such as pacing or electrophysiology electrodes. Preferably, the stimulation electrodes comprise large-area carbon electrodes, most preferably vitreous carbon, or alternatively, pyro-carbon. Both types of carbon materials are known for their compatibility with heart tissue, in-vivo durability and excellent electrical properties, including high electrical conductivity. Thus, they allow a relatively high electrical current to be delivered to a relatively large segment of the heart tissue, without inducing electrical excitation.

Alternatively, the non-excitatory stimulation electrodes may comprise large-arena electrodes of other types known in the art, such as platinum or platinum/iridium.

In other preferred embodiments of the present invention, the non-excitatory stimulation electrodes are inserted into one of the blood vessels of the heart, preferably into the coronary sinus, or alternatively, into a coronary artery. These preferred embodiments are based on the inventors' experimental findings that cardiac output is most enhanced when the stimulation electrodes are placed in the heart adjacent to the blood vessels. In one such preferred embodiment, the non-excitatory stimulation probe comprises a carbon wire electrode, which is inserted into the coronary sinus and passed therein to a position adjacent the left ventricle.

In some preferred embodiments of the present invention the stimulation probe comprises a plurality of stimulation electrodes. Preferably, the probe comprises a stimulation net, which includes a plurality of interconnected, individually- and/or collectively- addressable electrodes, covering a substantial segment of the heart wall. As described in the above-mentioned publication WO 9 725 098, the inventors have found that the extent of change in cardiac output, especially in left ventricular stroke volume, may be controlled by varying the size of the segment of the heart to which a non-excitatory field is applied. Such size variation is most preferably achieved by varying the number of the electrodes within the net that are to which the non-excitatory stimulation pulse is applied simultaneously.

In another preferred embodiment of this type, different stimulation pulses are applied to respective ones or groups of the plurality of stimulation electrodes. Preferably, the different stimulation pulses are applied to the respective electrodes with a predetermined delay between the different pulses. The delay may be varied so as to achieve a desired hemodynamic effect, for example, to maximize the increase in stroke volume.

In still other such preferred embodiments, the positions of the plurality of stimulation electrodes and/or characteristics of the stimulation pulses applied thereto are optimized responsive to clinical characteristics of the heart. Preferably, before insertion of the electrodes, a map of the heart is produced, for example, an electrophysiological map, as described in U.S. Patent 5,568,809 or a phase-dependent geometrical map, as described in PCT Patent publication WO 9 725 101. Preferably, the map includes information regarding the viability of the heart tissue, for example, based on local contractility or electrical activity. The non-excitatory stimulation electrodes are then positioned responsive to the map.

Alternatively or additionally, at the time of implantation of the non-excitatory stimulation electrodes, their positions are varied and the results of the variation on hemodynamics are observed, in order to find optimal, fixed positions for the electrodes. A similar effect may be attained by implanting the net electrode, as described above, and variably addressing different ones or groups of electrodes in the net so as to optimize the hemodynamic effect.

In alternative embodiments of the present invention, the non-excitatory stimulation probe comprises at least one hybrid electrode. The hybrid electrode preferably comprises a sensing core, preferably a platinum or platinum/iridium electrode, surrounded by an annular non-excitatory stimulation electrode, preferably comprising a carbon electrode, as described above.

In still other embodiments of the present invention, a single electrode may serve as both the sensing electrode and as one of the one or more stimulation electrodes.

In some preferred embodiments of the present invention at least one non-excitatory stimulation electrode and the sensing electrode are implanted in the same chamber of the heart, preferably in the left ventricle. Preferably, the non-excitatory stimulation electrode is fixed against the wall of the left ventricle, and the sensing electrode is fixed at the septum thereof Alternatively, the stimulation and sensing electrodes may be implanted in different chambers of the heart, in either the ventricles or the atria. The delay between the heart activity signal sensed by the sensing electrode and the pulse applied to the stimulation electrode is preferably dependent on the relative postions of these electrodes.

In some preferred embodiments of the present invention, the non-excitatory stimulation electrodes comprise a bipolar electrode, and the non-excitatory stimulation pulse is provided to the heart tissue between the poles of the electrode. In other preferred embodiments, the non-excitatory stimulation pulse is provided between the one or more stimulation electrode and another electrode, for example, the sensing electrode. Alternatively, the pulse may be applied between the stimulation electrodes and the case of the control circuitry, in those embodiments of the present invention in which the case is implanted in the patient's body, as described above.

In preferred embodiments of the present invention, the control circuitry applies to the stimulation electrodes a square wave stimulation pulse having current up to 50 mA, most preferably between 5 and 10 mA, for a duration that may be nearly as long as the beat-to-beat interval of the heart, most preferably between 30 and 80 msec. Preferably, the stimulation pulse is followed by a pulse of opposite polarity, to prevent problems of electrode degradation and polarization.

In some preferred embodiments of the present invention, an alternating waveform is superimposed on the square wave pulse. The waveform is itself preferably a square wave or, alternatively a sinusoidal or a sawtooth wave, having a frequency up to 100 kHz and amplitude less than or comparable to that of the square wave pulse. The inventors have found that in at least some cases, such a waveform enhances the hemodynamic effect of the non-excitatory stimulation pulse on the heart muscle.

In some preferred embodiments of the present invention, the control circuitry also receives a sensor signal indicative of hemodynamic conditions, preferably indicative of the cardiac output, and adjusts the stimulation pulse responsive to the signal to achieve a desired cardiac output level. Alternatively or additionally, physiological sensors may be used to sense other hemodynamic parameters, such as blood pressure or blood oxygenation, so as to provide feedback signals to the control circuitry or to telemetry apparatus associated with the control circuitry. Sensors used for this purpose may thus include flow rate sensors, pressure sensors, temperature sensors, oxygen sensors, and other types of sensors known in the art. The control circuitry then adjusts the stimulation pulse so that the hemodynamic parameters are maintained within a desired range of values.

Although preferred embodiments of the present invention are described for the most part with reference to sensing electrogram signals in the heart, so the cardiac output controller applies the non-excitatory stimulation pulse in response to and generally synchronized with the sinus rhythm, cardiac output controllers in accordance with the present invention may also be synchronized by other methods For example, as described above, the non-excitatory stimulation pulse may be synchronized relative to a body-surface ECG, and may also be synchronized and controlled relative to an arrhythmic heart beat.

Alternatively, the pulse may be externally synchronized, for example by an externally-applied trigger pulse or by a pacing pulse that is applied to the heart. These aspects of the present invention are further described in a PCT patent publication WO 9 810 832.

Preferred embodiments of the present invention may also be used in conjunction with suitable drugs, as described in a PCT patent publication entitled "Drug-Device Combination for Controlling the Contractility of Muscles" (WO 9 810 829), and in conjunction with devices and methods for preventing cardiac fibrillation, as described in a PCT patent publication. entitled "Fencing of Cardiac Muscles" (WO 9 810 830).

There is therefore provided, in accordance with a preferred embodiment of the present invention, an apparatus for modifying cardiac output of the heart of a subject, including:
one or more sensors, which sense signals responsive to cardiac activity;
a stimulation probe including one or more stimulation electrodes, which apply non-excitatory stimulation pulses to a cardiac muscle segment; and
signal generation circuitry, coupled to the one or more sensors and the stimulation probe, which circuitry receives the signals from the one or more sensors and generates the non-excitatory stimulation pulses, responsive to the signals.

Preferably, the signal generation circuitry is contained in a unit external to the subject's body, or alternatively, in an implantable case

Preferably, application of the stimulation pulses engenders an increase in the cardiac output, or alternatively, a decrease in the cardiac output.

Alternatively or additionally, application of the stimulation pulses increases an efficiency of contraction of the heart.

Preferably, the one or more sensors include a intracardiac electrode, and the signal generation circuitry synchronizes the stimulation pulse to electrical activity of the heart. Alternatively, the one or more sensors includes a body surface electrode, and the signal generation circuitry synchronizes the stimulation pulse to an ECG signal. Preferably, the signal generation circuitry identifies an arrhythmia in the signals and controls the stimulation pulses responsive thereto. Additionally or alternatively, the signal generation circuitry detects a QT interval in the signals and controls the stimulation pulses responsive thereto.

Preferably, the signal generation circuitry varies one or more parameters of the stimulation pulse, from the group of parameters including voltage, current, duration, timing delay, waveform and waveform frequency. After the non-excitatory stimulation pulse, the signal generation circuitry generates another pulse of opposite polarity to the stimulation pulse, which is applied to the cardiac muscle segment by the stimulation probe.

Preferably, the one or more stimulation electrodes apply the stimulation pulse to a heart segment having an area of at least 5 mm², more preferably at least 1 cm², and most preferably at least 4 cm².

In a preferred embodiment of the invention, the signal generation circuitry varies the area of the heart segment to which the stimulation pulse is applied. Preferably, the stimulation probe includes a net of electrodes, which are addressable such that an extent of the segment to which the stimulation pulses is applied is controlled by addressing selected electrodes in the net. Preferably, the circuitry applies multiple, different stimulation pulses, preferably a time sequence of pulses, to different ones of the electrodes in the net.

In another preferred embodiment of the invention, the stimulation probe includes a hybrid electrode, including the intracardiac electrode together with at least one of the one or more stimulation electrodes. Preferably, the hybrid electrode includes a core section including the sensing electrode, enclosed within an annular section including the at least one stimulation electrode, wherein the annular section preferably includes a carbon material.

In still another preferred embodiment, the one or more stimulation electrodes include an elongate electrode, which is inserted into a blood vessel of the heart.

Preferably, the one or more stimulation electrodes include vitreous carbon or, alternatively, pyrolitic carbon.

In a preferred embodiment of the invention the one or more sensors include a hemodynamic sensor, which generates signals responsive to a hemodynamic parameter. Preferably, the hemodynamic sensor generates the signals responsive to blood flow. Alternatively or additionally, the hemodynamic sensor generates the signals responsive to blood oxygenation and/or temperature Preferably, the one or more sensors include an electrode, which senses cardiac electrical activity.

Preferably, the apparatus includes a telemeny device, which receives the signals from at least one of the one or more sensors and controls the signal generation circuitry to adjust the non-excitatory stimulation pulse.

There is further provided, not forming part of the present invention, a method for modifying cardiac output, including:
applying a stimulation probe including one or more stimulation electrodes to a subject's heart;
receiving a signal from at least one sensor responsive to the subject's cardiac muscle activity;
generating a non-excitatory stimulation pulse responsive to the signal and conveying the pulse to at least one of the one or more electrodes.

Preferably, receiving the signal includes introducing a sensing electrode into the heart and receiving signals therefrom, and generating the stimulation pulse includes generating a pulse synchronized with electrical activity sensed by the sensing electrode.

Alternatively or additionally, receiving the signal includes applying an electrode to a body surface and receiving signals therefrom, and wherein generating the stimulation pulse includes generating a pulse synchronized with an ECG signal.

Further alternatively or additionally, receiving the signal includes receiving signals from at least one of the one or more stimulation electrodes.

Preferably, generating the stimulation pulse includes generating a pulse having a predetermined delay relative to the signal.

Applying the stimulation probe includes applying a probe including a plurality of stimulation electrodes, and generating and conveying the pulse includes generating a sequence of pulses and applying each pulse in the sequence to a different one of the plurality of stimulation electrodes.

Preferably, generating and conveying the stimulation pulse includes generating and conveying stimulation pulses selectively, based on a characteristic of the signals received from the at least one sensor Further preferably, generating and conveying the pulses includes generating and applying pulses at a rate dependent on the heart rate, but not equal to the heart rate. Alternatively or additionally, generating and conveying the pulses includes detecting a cardiac arrhythmia and adjusting the application of the pulses responsive thereto. Further additionally or alternatively, generating and conveying the pulses comprises detecting a QT interval in the signals and generating pulses responsive thereto.

Preferably, generating the non-excitatory stimulation pulse includes varying one or more parameters of the pulse, selected from the group including the pulse voltage, current, duration, delay, waveform and waveform frequency.

Further preferably, the pulse includes a baseline pulse and a waveform, preferably a square wave, of substantially higher frequency than the baseline pulse superimposed thereon. Preferably, after generating the non-excitatory stimulation pulse, another pulse of opposite polarity thereto is generated and conveyed to the electrodes.

Applying the non-excitatory stimulation pulse includes varying the extent of a portion of the area of the heart segment to which the stimulation pulse is applied, wherein varying the extent preferably includes selectively addressing a net of stimulation electrodes implanted in the heart.

Applying the stimulation probe includes inserting the one or more stimulation electrodes in multiple chambers of the heart.

Implanting the stimulation probe includes inserting at least one of the one or more stimulation electrodes into a blood vessel of the heart, preferably into the coronary sinus

Receiving the signal includes sensing a hemodynamic parameter, preferably sensing cardiac output, wherein generating the pulse preferably includes changing the pulse responsive to the signal to increase the cardiac output, or alternatively, to decrease the cardiac output.

Alternatively or additionally, sensing the hemodynamic parameter includes sensing a pressure and/or a flow rate and/or oxygenation and/or a temperature.

Generating and conveying the pulse includes generating and conveying pulses at selected times of day, preferably conveying pulses to increase cardiac output during the subject's waking hours.

Preferably, generating and conveying the pulses includes generating and conveying pulses which increase the subject's cardiac output, or alternatively decrease the subject's cardiac output. Further alternatively or additionally, generating and conveying the pulses includes generating and conveying pulses which increase the efficiency of contraction of the heart.

Preferably, conveying the pulses includes applying pulses to a heart segment having an area of at least 5 mm², more preferably at least 1 cm², and most preferably at least 4 cm².

The present invention will be more fully understood from the following detailed description of the preferred embodiments thereof, taken together with the drawings in which:

### BRIEF DESCRIPTION OF THE DRAWINGS

Fig. 1A is a schematic illustration showing a device for controlling cardiac output, in accordance with a preferred embodiment of the present invention.
Fig. 1B is a schematic illustration showing a miniaturized implantable device for controlling cardiac output, in accordance with an alternative preferred embodiment of the present invention,
Fig. 2A is a schematic sectional illustration showing the heart of a patient, into which stimulation and sensing electrodes for use in conjunction with the device of Fig. 1A are inserted, in accordance with a preferred embodiment of the present invention;
Fig. 2B is a schematic illustration of a non-excitatory stimulation probe, in accordance with another preferred embodiment of the present invention;
Fig. 3 is a schematic block diagram of control circuitry use in the devices depicted in Fig. 1A, in accordance with a preferred embodiment of the present invention;
Fig. 4 is a flow chart illustrating a method of regulating cardiac output;
Fig. 5 is a schematic illustration showing a square wave stimulation pulse applied by stimulation electrodes to the patient's heart, in accordance with a preferred embodiment of the present invention;
Fig. 6 is a schematic illustration of a stimulation electrode for use in conjunction with the devices of Figs. 1A and 1B, in accordance with a preferred embodiment of the present invention;
Fig. 7 is a schematic illustration of a hybrid electrode for use in conjunction with the devices of Figs. 1A and 1B, in accordance with an alternative preferred embodiment of the present invention; and
Figs. 8-32 are electronic schematic electronic diagrams showing circuitry for use in the device of Fig. 1A, in accordance with a preferred embodiment of the present invention.

### DETAILED DESCRIPTION OF PREFERRED EMBODIMENTS

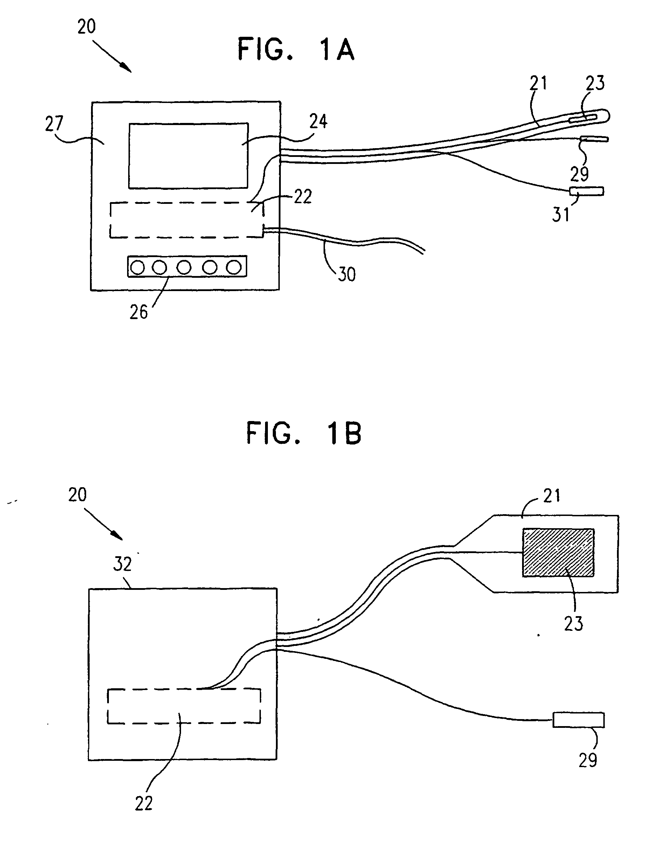

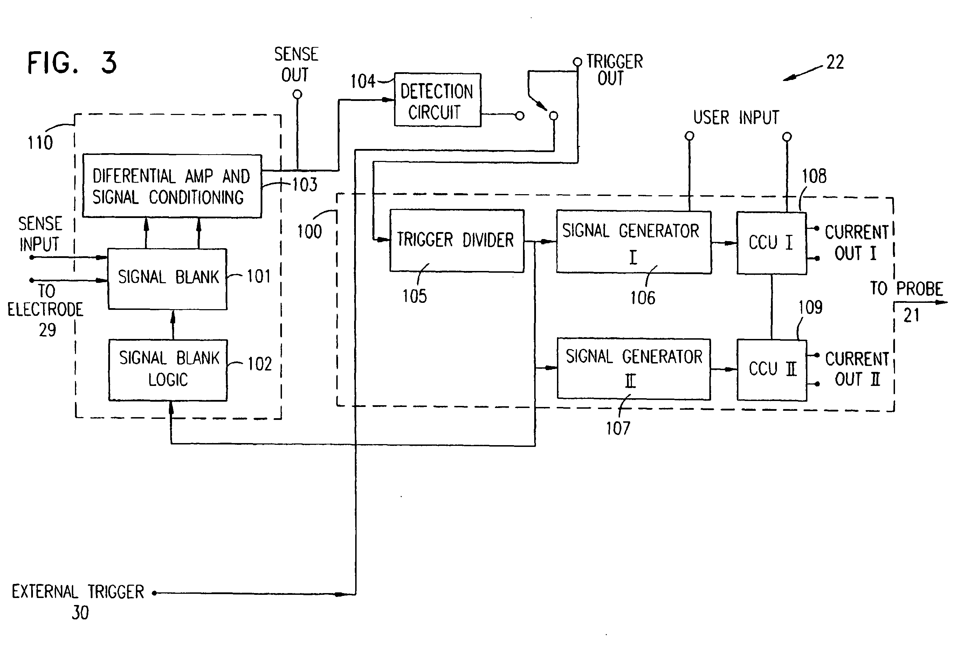

Fig. 1A schematically illustrates a device 20 for regulating cardiac output, comprising a control unit 27, an implantable non-excitatory stimulation probe 21, including at least one non-excitatory stimulation electrode 23, and an implantable sensing electrode 29. Control unit 27 includes signal generation circuitry 22 (shown in Fig. 3 below and described with reference thereto), as well as an optional display 24 and a control panel 26. When properly implanted in the heart, as described below, sensing electrode 29 receives local electrogram signals from the heart muscle and transmits them to the signal generation circuitry 22 The circuitry generates a non-excitatory stimulation pulse responsive to the electrogram signal, which pulse is applied by probe 21 to cardiac muscle tissue. Control unit 27 optionally also includes an external trigger input 30. Device 20 may further includes another physiological sensor 1, for example, a flow sensor, for determining the effect of the stimulation provided by the device on the cardiac output.

Fig. 1B schematically illustrates device 20 in accordance with an alternative preferred embodiment of the present invention, in which signal generation circuitry 22 is contained in an implantable miniaturized electronic control circuitry case 32, similar to implantable pacemaker cases known in the art. Unlike control unit 27, case 32 does not include display 24 and control panel 26, but in other respects, the embodiment of Fig. 1B is functionally similar to that of Fig. 1A.

In the embodiment shown in Fig. 1B, stimulation electrode 23 is shown to be a relatively large area electrode, designed to provide stimulation an area of at least 5 mm² in the heart. Electrode 23 preferably comprises a low-resistance carbon material, preferably vitreous carbon, or alternatively, pyrolitic carbon. Electrodes made of such materials are manufactured, for example, by Sorin Biomedica, of Saluggia, Italy, and by Goodfellow Cambridge Ltd., of London, England. Both types of carbon materials are known for their compatibility with heart tissue, in-vivo durability and excellent electrical properties. It will be understood, however, that other types of electrode materials made also be used, such as various types described in *A Guide to Cardiac Pacemakers, Defibrillators and Related Products*, by Morse, et al. (Droege Computing Services, Durham, North Carolina). Stimulation probe 21 may comprise more than a single stimulation electrode. Sensing electrode 29 may comprise any suitable type of intracardiac electrode, known in the art.

Preferably, electrodes 23 and 29 are coated with an anticoagulant, such as heparin, preferably in a time-release form, or elute the anticoagulant into the heart tissue, to prevent clot formation on and around the electrodes. Such electrodes may be produced in a manner similar to steroid-eluting electrodes known in the art, for example, the Medtronic CAPSURE model 4003 electrode, described in *The Foundations of Cardiac Pacing*, by Sutton and Bourgeois, p. 73.

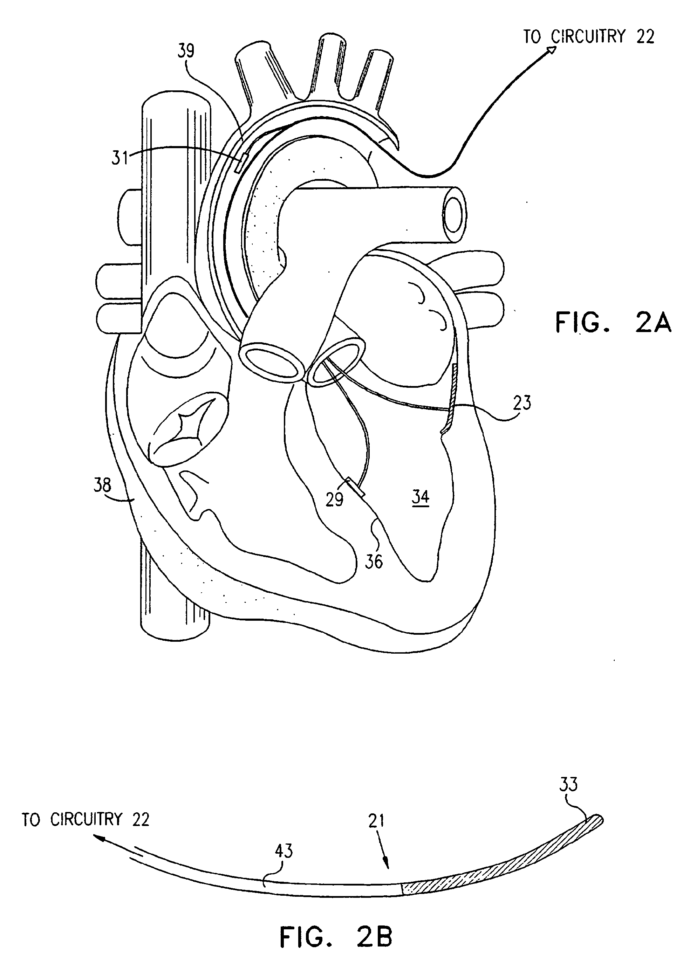

Fig. 2A is a schematic illustration showing electrodes 23 and 29 implanted in a subject's heart 38, in accordance with a preferred embodiment of the present invention. Electrodes 23 and 29 are implanted in a chamber of the heart, preferably both in left ventricle 34. Most preferably, non-excitatory stimulation electrode 23 is implanted in contact with the wall of left ventricle 34, and sensing electrode 29 is implanted in septum 36, between the ventricles. Electrodes 23 and 29 are connected by wires passing through aorta 39 to either control unit 27 outside the body or to implantable case 32, which is preferably implanted in the patient's chest. When circuitry 22 detects an electrical activation pulse in the electrogram signal received from electrode 29, it generates a stimulation pulse, which is applied to electrode 23, preferably so as to increase the contraction of at least the segment of the wall of ventricle 34 with which electrode 23 is in contact, thus increasing the ventricular stroke volume.

Alternatively, stimulation electrode 29 and, optionally, sensing electrode 23 may be implanted surgically, preferably in the epicardium.

Fig. 2B is a schematic illustration showing an alternative preferred embodiment of the present invention, in which non-excitatory stimulation probe 21 comprises a wire electrode 33. The electrode, which preferably comprises carbon material, as described above, is connected by an insulated conductor 43 to circuitry 22. It is preferably implanted in one of the blood vessels of the heart. Most preferably, electrode 33 is passed through the right atrium of the heart, into the coronary sinus, using techniques of catheterization known in the art, and is positioned therein adjacent to left ventricle 34. The electrode is then driven by circuitry 22 to deliver the non-excitatory stimulation pulses to the heart wall, as described herein.

Fig. 3 is a schematic block diagram of signal generation circuitry 22, in accordance with a preferred embodiment of the present invention Circuitry 22 comprises a stimulation section 100, a detection circuit 104 and a sensing unit 110. Unit 110 receives and conditions electrogram signals from sensing electrode 29. Detection circuit 104 senses a local activation wave in the electrogram, preferably by detecting a slope and voltage level corresponding to the rising edge of the wave, as is known in the art, and generates a trigger pulse responsive thereto. The trigger pulse is conveyed to stimulation section 100, which generates the stimulation pulses and applies these pulses to the electrodes of stimulation probe 21.

Sensing unit 110 includes signal blanking unit 101 and signal blank logic 102 and a differential amplifier and signal conditioning circuit 103. The blanking operates to block the input to detection circuit 104 while the output of stimulation section 100 is active, to prevent the system from generating trigger pulses due to stimulation artifacts.

Stimulation section 100 comprises a trigger divider 105, which generates a modified trigger pulse in response to input trigger pulses from detection circuit 104 or alternatively from external trigger input 30. The trigger divider allows a user of device 20 to select whether the stimulation pulse will be applied at every heart beat or only once in a predetermined number of beats. Section 100 further includes signal generators 106 and 107, which generate voltage signals of predefined characteristics, as described below, in response to the modified trigger pulse, and constant current units (CCU) 108 and 109, which convert input voltage signals from the signal generators to output current pulses. Two stimulation output channels are shown in Fig. 3, enabling different stimulation pulses to be applied to two or more different stimulation electrodes. It will be appreciated, however, that only one of the channels need be used or alternatively, that additional channels may be added to drive additional stimulation electrodes.

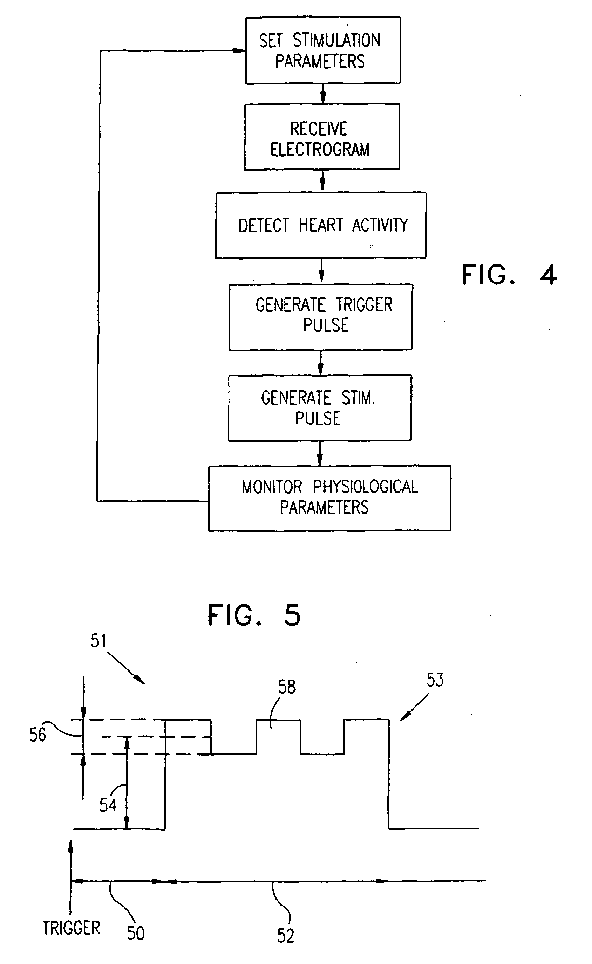

Fig. 4 is a flow chart, which summarizes a method for regulation of cardiac output using device 20, not forming part of the present invention. Preferably, parameters of the stimulation pulse, such as its level, duration, delay and waveform characteristics, as described below, are preset by the user. Sensing electrode 29 senses electrogram signals, as described above, and signal generation circuitry 22 receives these signals and inputs them to detection circuit 104, which detects heart activity, preferably the sinus rhythm, and generates a trigger pulse responsive thereto. The trigger pulse drives stimulation section 100 to generate the stimulation pulse, which is applied to the heart by stimulation electrode 23. In an alternative preferred embodiment, an external trigger coupled to input 30 is employed.

Optionally, physiological parameters related to the cardiac output are monitored in order to verify the efficacy of the non-excitatory Stimulation. For example, flow of blood through aorta 39 may be detected by flow sensor 31, as illustrated in Fig. 2A. Preferably, at least some of the parameters of the stimulation pulse, for example, its amplitude and/or timing, are adjusted responsive to the monitored parameters so as to achieve a desired cardiac output level. Such monitoring and adjustment are preferably carried out on-line, for example, by control unit 27 itself. Alternatively or additionally, a separate telemetry unit (not shown in the figures) monitors the parameters and is used two program control unit 27 to vary the parameters of the stimulation pulse accordingly.

Fig. 5 is a schematic illustration of a non-excitatory stimulation pulse 51 applied by stimulation electrode 23 to cardiac tissue, in accordance with a preferred embodiment of the present invention. In some preferred embodiments of the present invention the regulation of cardiac output is achieved by varying certain characteristics of pulse 51. Non-excitatory stimulation energy is applied to stimulation electrode 23 in the form of a baseline pulse 53 having a baseline amplitude, indicated by an arrow 54 in Fig. 5, of preferably 5 to 10 mA, optionally up to 50 mA. The duration of pulse 51, indicated by an arrow 52, preferably ranges between 30 and 80 ms, and optionally up to 500 ms. Pulse 53 is preferably followed by another pulse of opposite polarity (not shown in the figure) to prevent problems of tissue polarization and electrode degradation, as described in the above-referenced PCT publication WO 9 725 098 and mentioned above. Preferably, a waveform 58 having a frequency of up to 10 kHz and amplitude, indicated by an arrow 56, up to or comparable to the baseline amplitude is superimposed on the baseline amplitude of pulse 53 Although waveform 58 is shown here as a square wave, any other suitable waveform may be used, for example, a sinusoid or sawtooth wave.

Preferably, waveform 51 is triggered upon the detection of the rising edge of the R-wave of the heart's electrical activity by detection circuit 104. Alternatively, signal generators 106 and 107 may be controlled to provide a delay, indicated by an arrow 50 in Fig. 5, of between 1 and 500 msec between the trigger input and the pulse generation. The appropriate delay may depend on whether the trigger is provided by detection circuit 104 or by external trigger 30, as well as on the relative positions of sensing electrode 29 and sensing electrode 23. The delay is adjusted based on the desired increase or decrease of cardiac output that is to be achieved, and the optimal delay will generally vary from patient to patient.

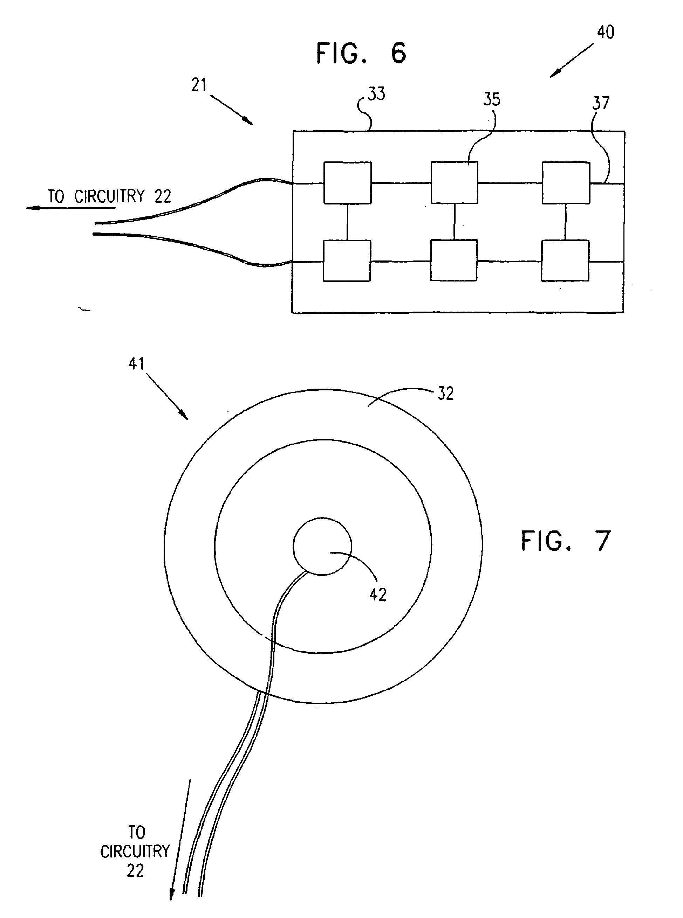

Fig. 6 is a schematic illustration showing an electrode net 40, for use as part of stimulation probe 21, in accordance with a preferred embodiment of the present invention. Net 40 comprises a plurality of stimulation electrodes 35, preferably, interconnected by a conductor network 37. Electrodes 35 are preferably individually and/or collectively addressable, and may operate in either a unipolar or a bipolar mode Net 40 is preferably large enough to cover a substantial segment of the heart wall, preferably at least 1 cm² and most preferably at least 4 cm². Each individual electrode 35 preferably has an area of at least 5 mm², and is separated from its neighbors preferably by at least 1 cm.

In this preferred embodiment, cardiac output regulation is preferably achieved through variation of the stimulated area of a segment of the heart wall with which net 40 is in contact, as described in the '012 PCT patent application. Preferably, the stimulated area is varied by changing the active area of net 40, i.e., varying the extent of the area of the net over which electrodes 35 are driven to deliver electric stimulation to the heart. The inventors have found, for example, that when the non-excitatory stimulation is applied between pairs of electrodes, selected among a plurality of electrodes placed in the left ventricle, the resultant enhancement of left-ventricular pressure and cardiac output varies responsive to the relative positions of the electrodes in the pair and the distance between them Electrodes 35 in net 40 may thus be selectably addressed to optimize the hemodynamic results of the stimulation. Moreover, electrodes 50 may also be used as sensing electrodes, to map cardiac electrical activity over the segment of the heart wall, so that the stimulation may be applied responsive to the map.

Fig. 7 schematically illustrates a hybrid electrode probe 41 for use with device 20, in accordance with an alternative preferred embodiment of the present invention. Probe 41 comprises an annular stimulation electrode 32, preferably a carbon electrode, as described above, surrounding a smaller sensing electrode 42 at the center of the probe, preferably, a platinum or platinum/iridium electrode, most preferably bipolar. It will be understood that the methods, stimulation waveforms and control electronics described above in relation to electrode 23 may be applied using probe 41, as well as any other suitable electrode configuration. Probe 41 is advantageous in that it reduces the number of separate electrodes that need to be introduced into and implanted in the heart.

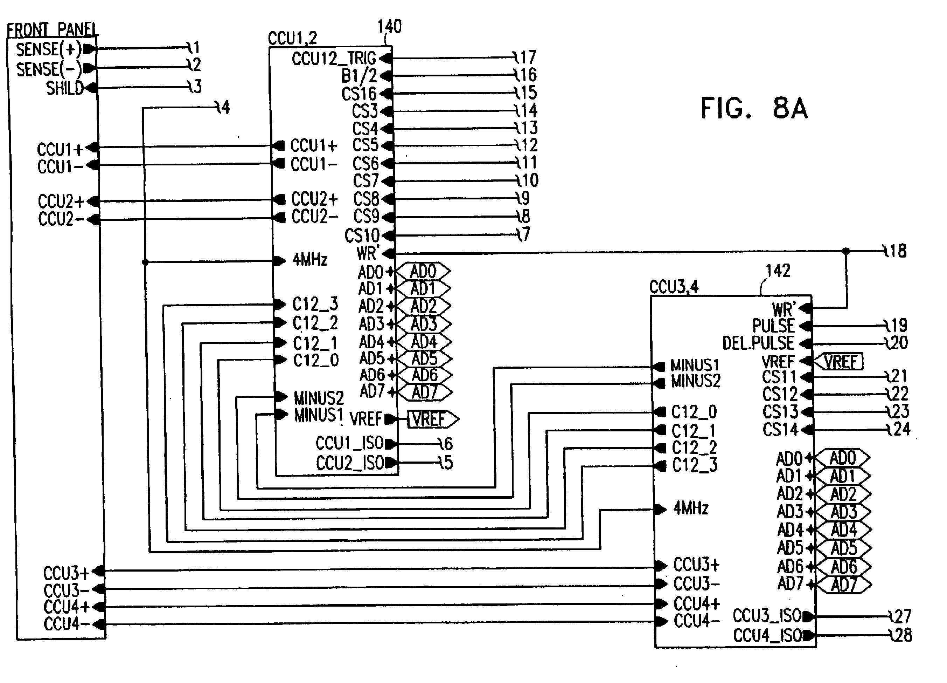

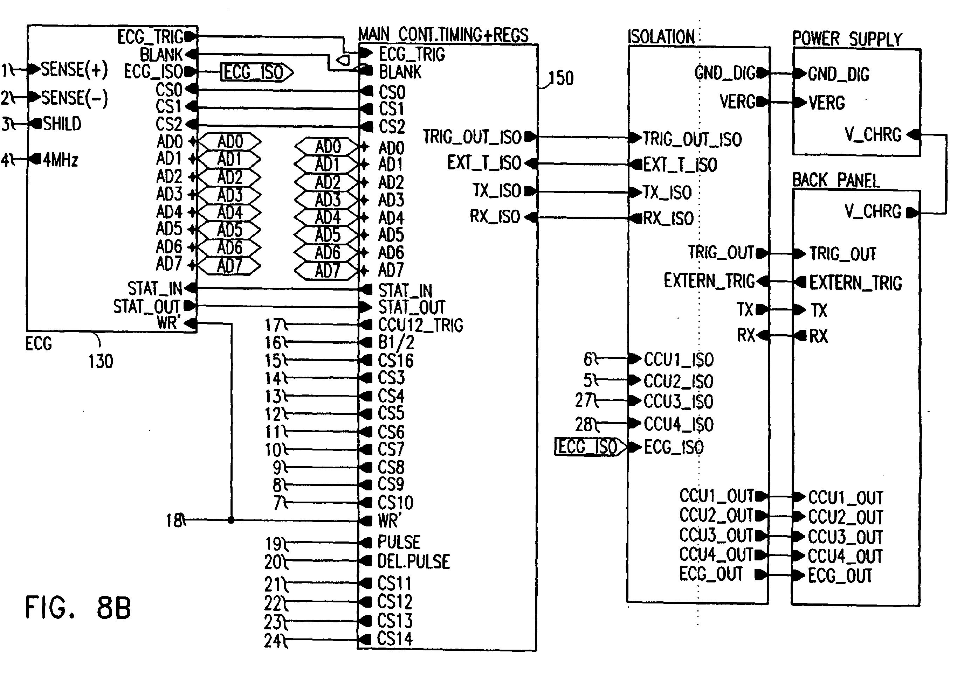

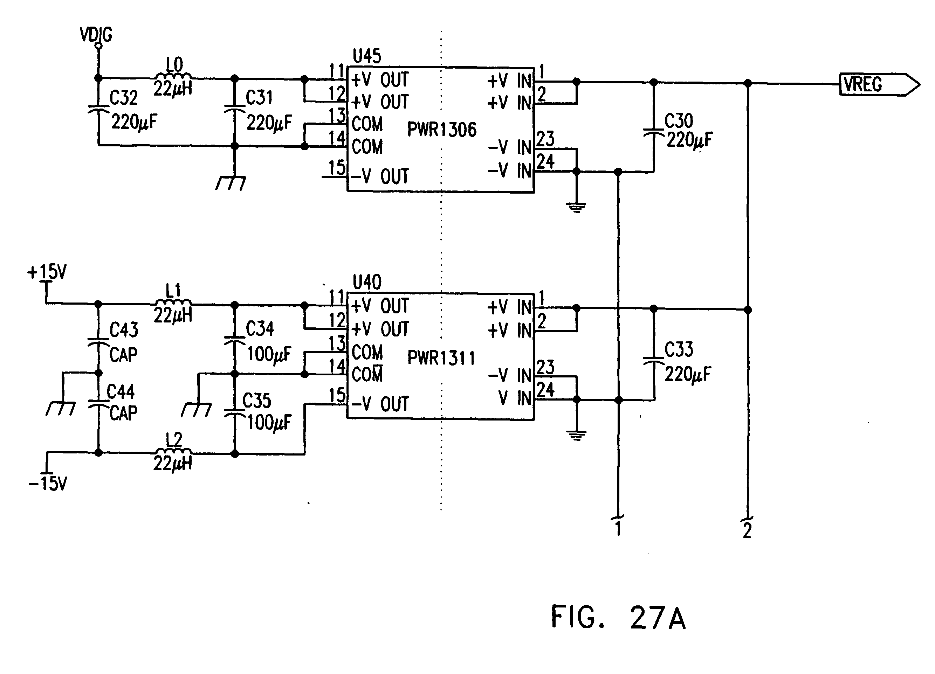

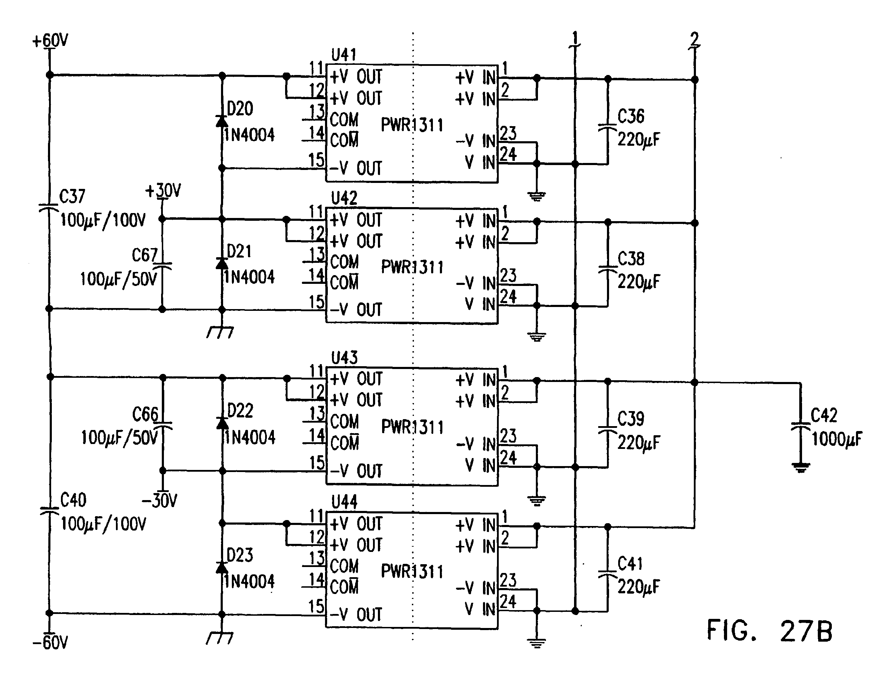

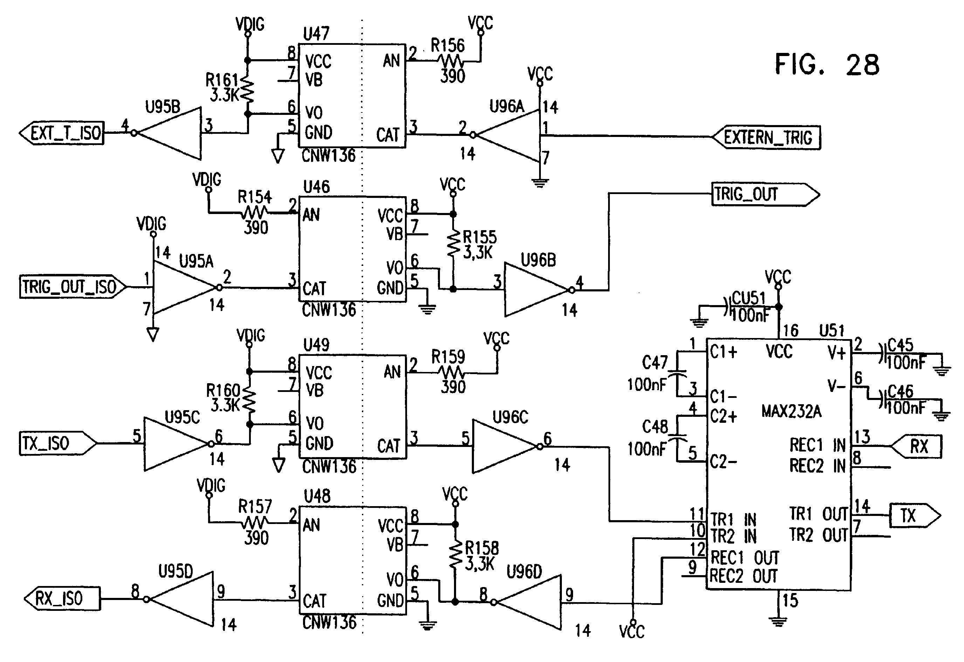

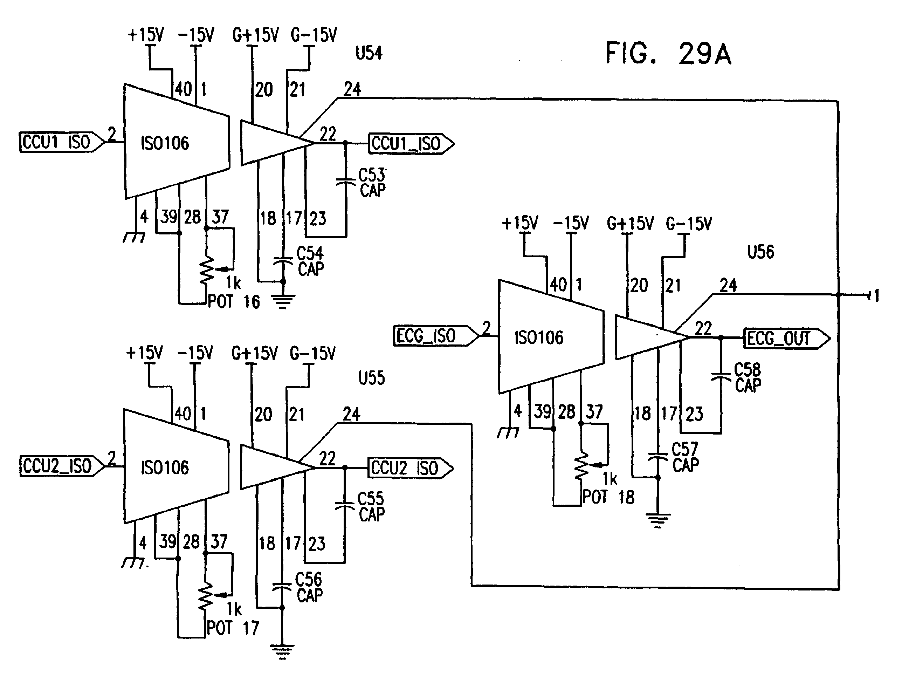

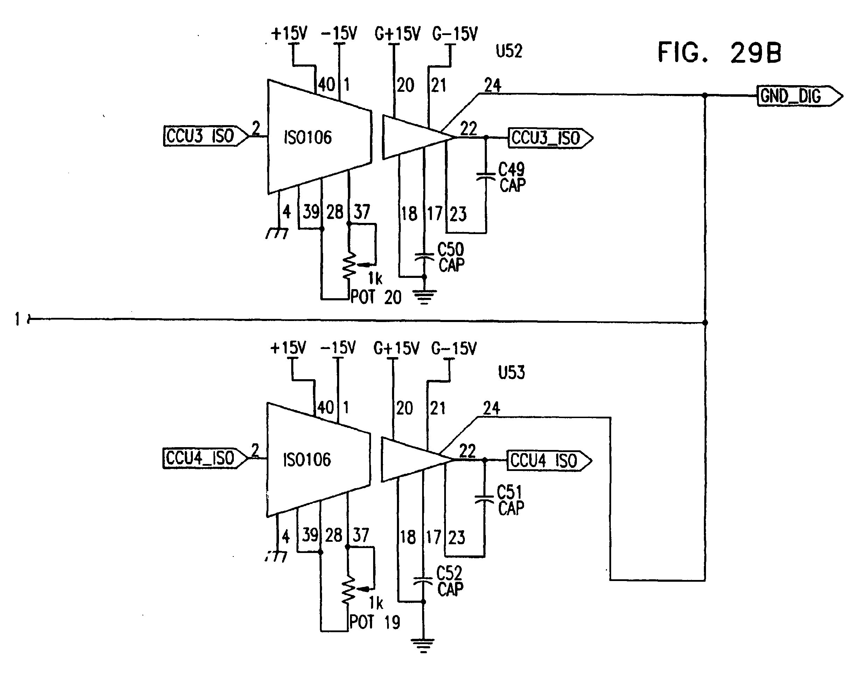

Figs. 8-31 are electronic schematic diagrams illustrating circuitry for use in implementing the functions of circuitry 22, in accordance with a preferred embodiment of the present invention. As shown in Figs. 8A and 8B, the circuitry includes an ECG processor 130, a first CCU section 140, and main control circuit 150, which together perform the functions of circuitry 22, as shown in Fig. 3 and described with reference thereto. In addition, a second CCU section 142 is designed to provide, optionally, excitatory stimulation pulses, i.e., to pace the heart. This element is beyond the scope of the present invention, however, and is described in detail in the above-mentioned PCT patent publication WO 9 810 832 entitled "Cardiac Output Enhanced Pacemaker".

Figs. 9 through 3 are circuit diagrams showing details of the implementation of the elements of Figs. 8A and 8B. These diagrams are considered sufficient in and of themselves to enable one skilled in the art to practice the present invention. Various aspects of these diagrams are described hereinbelow.

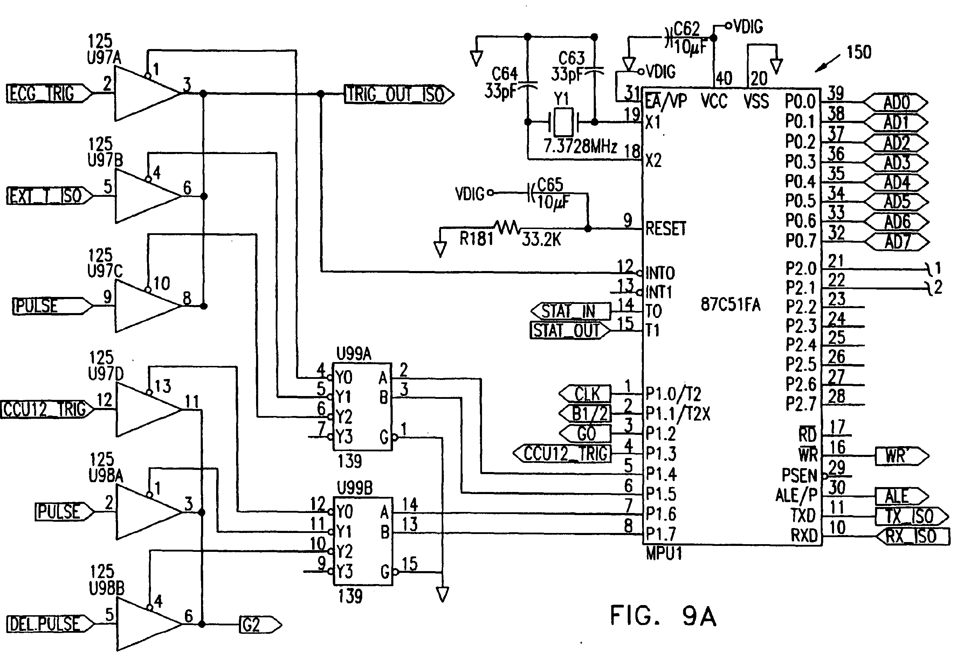

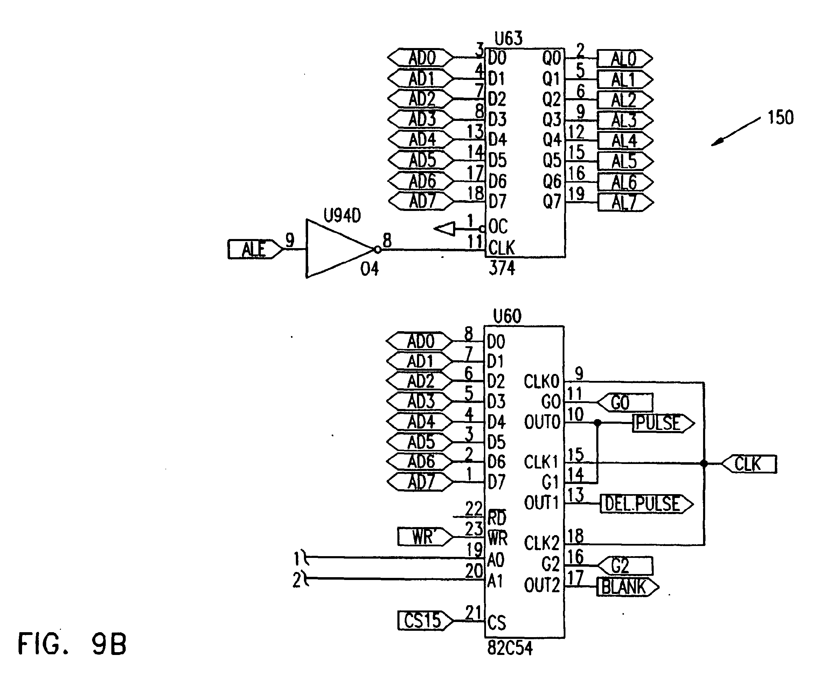

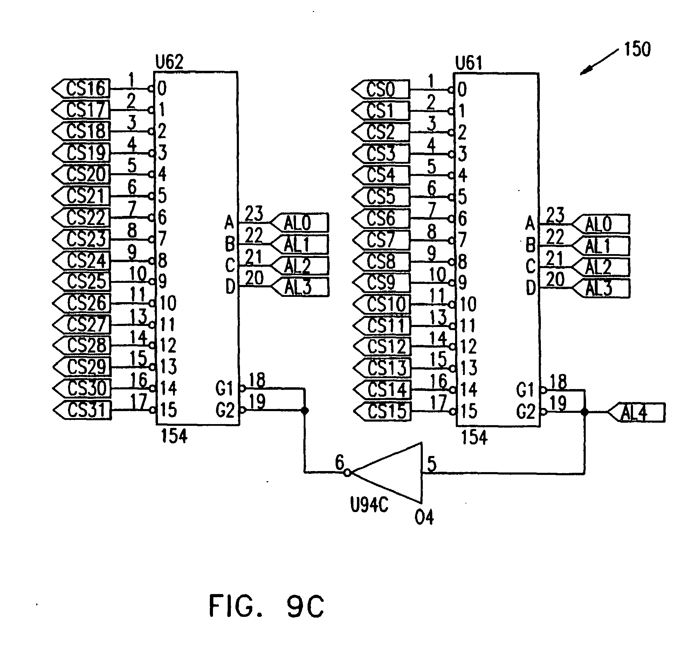

Figs. 9A, 9B and 9C illustrate main control circuit 150, which is based on a microcontroller MPU1, preferably an 8051-type microcontroller, as is known in the art. The microcontroller receives user commands via a communications interface, for example, to program parameters of the stimulation pulses to be applied. It controls other elements of circuitry 22 via a data bus, marked AD0-AD7.

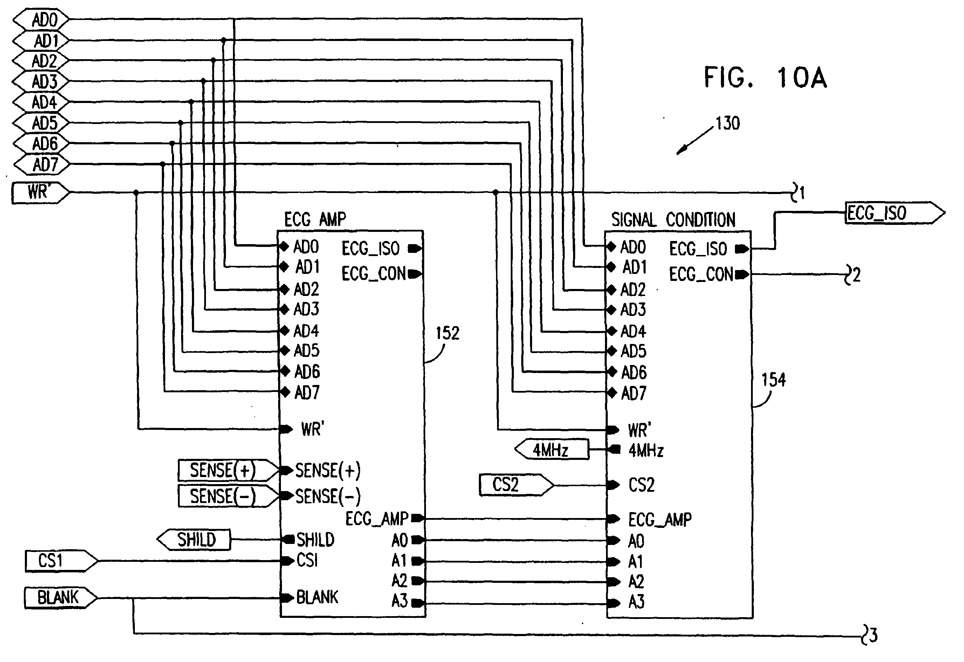

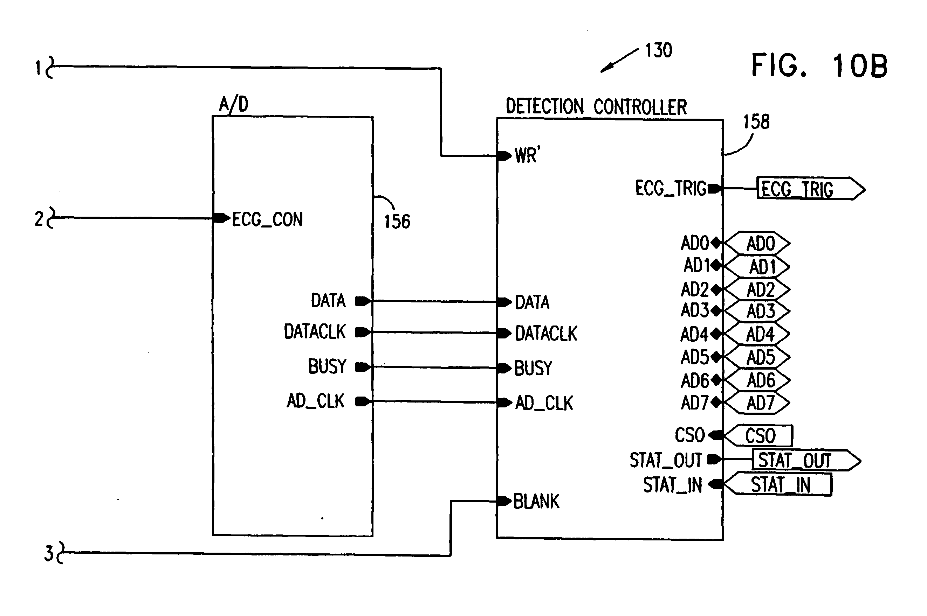

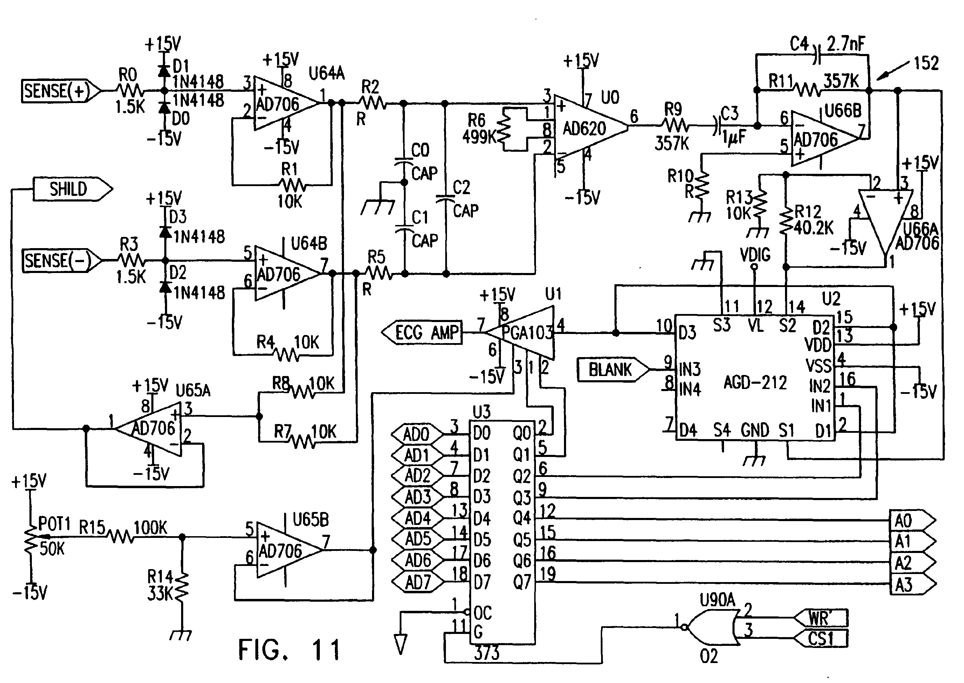

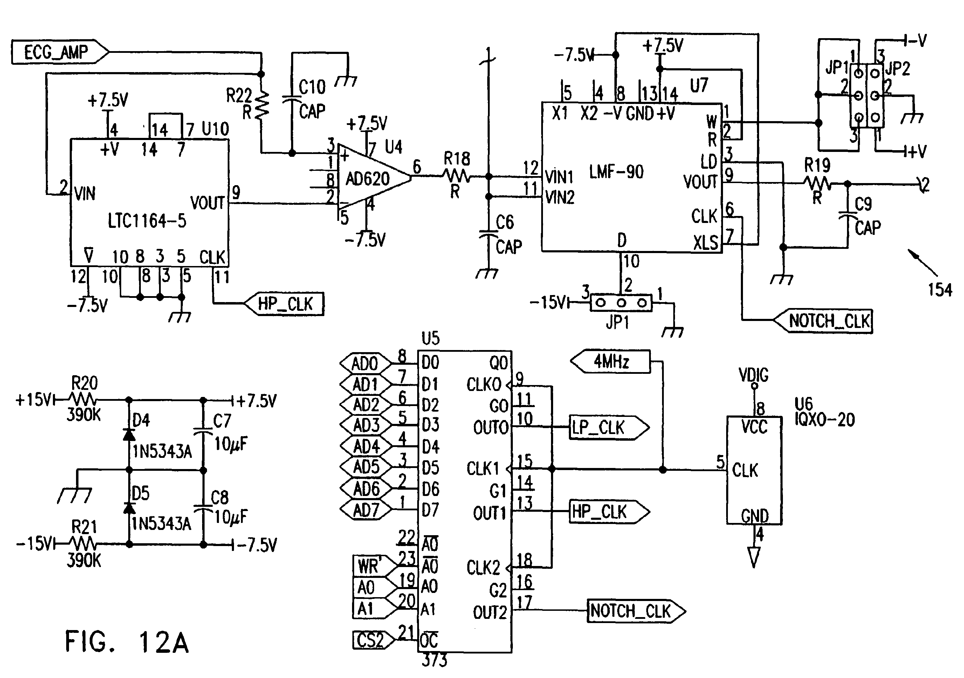

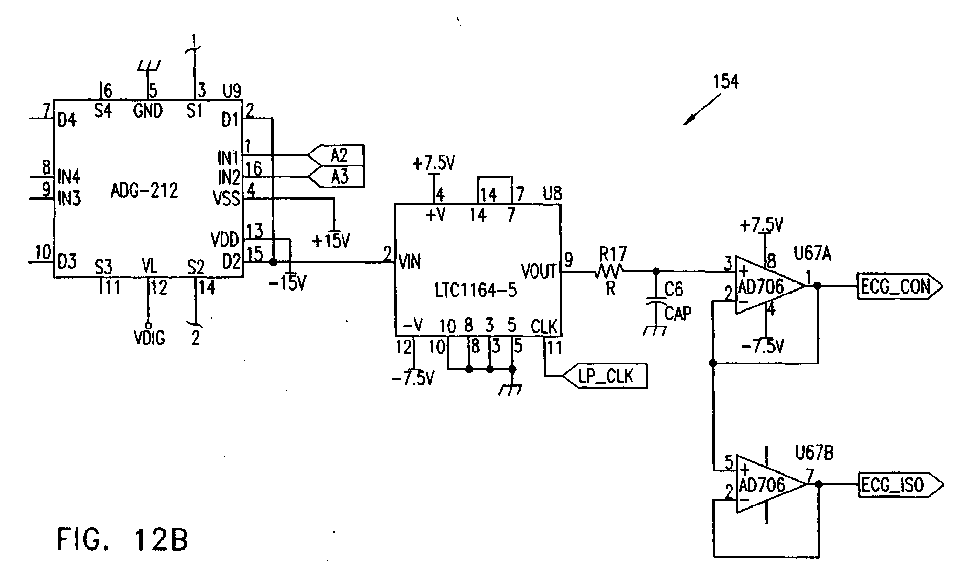

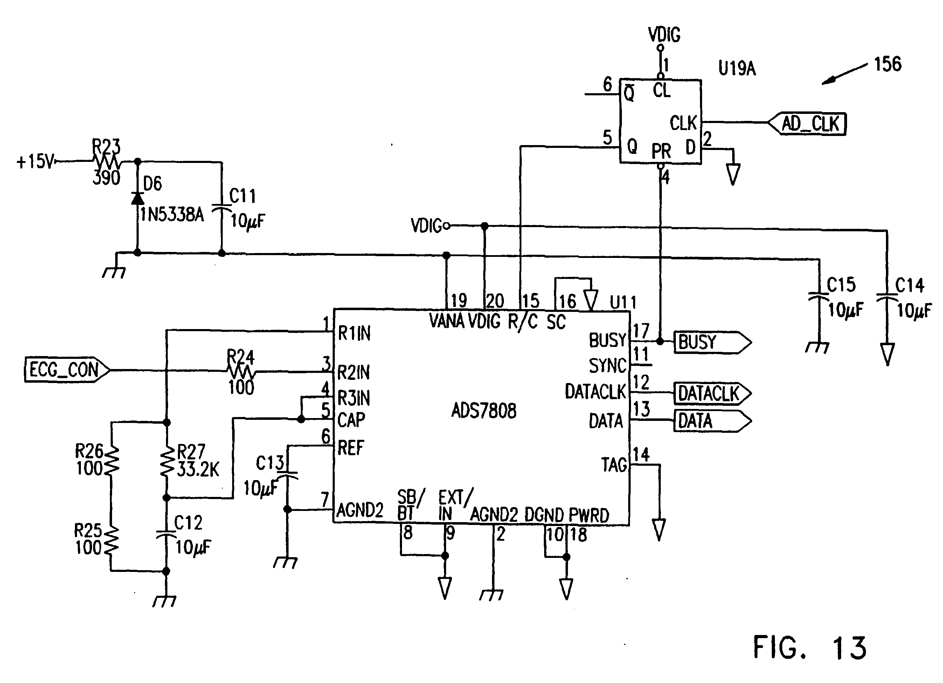

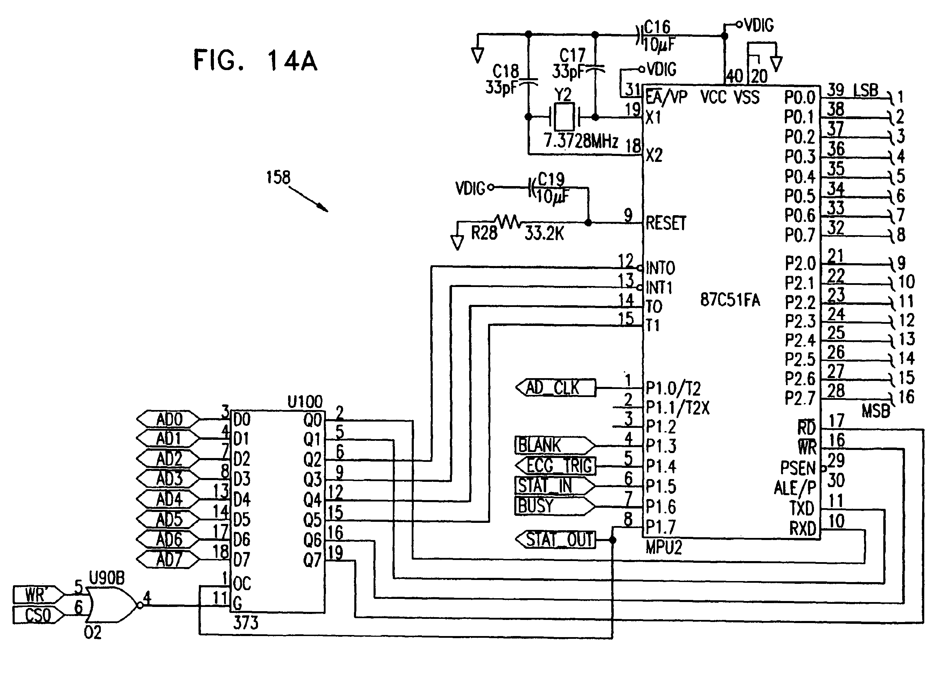

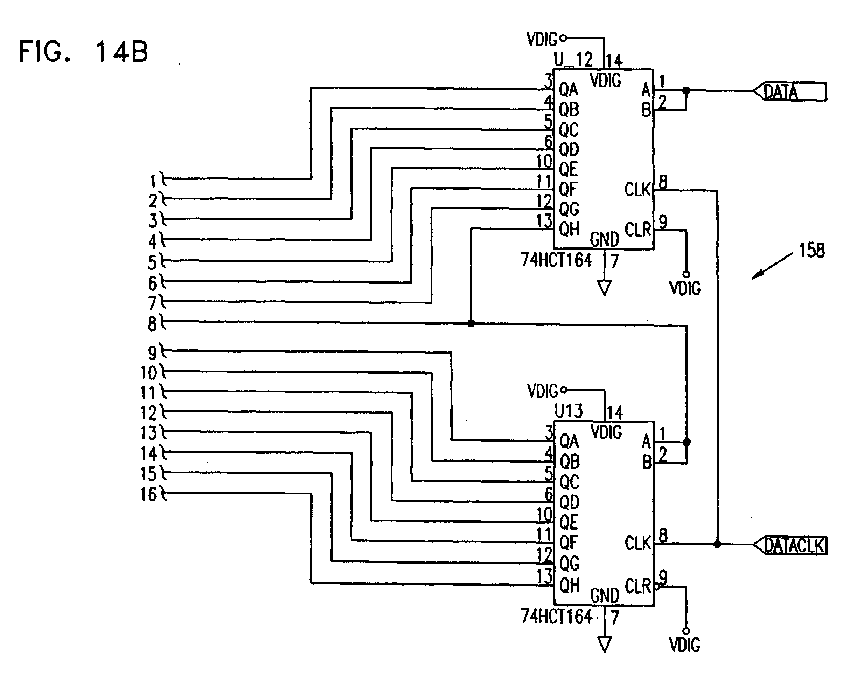

Figs. 10A and 10B show details of ECG processor 130, which receives electrical signals from the patient's body and processes them to generate trigger pulses, as described above, for driving the non-excitatory stimulation ECG processor 130 includes an ECG amplifier 152, an ECG signal conditioning unit 154, an A/D converter 156, and a detection controller 158. ECG amplifier 152 is shown in detail in Fig. 11, and comprises a differential preamplifier and programmable gain amplifier and banking unit. Signal conditioning unit 154, shown in Figs. 12A and 12B, includes programmable high-pass, low-pass and notch filters, selectable by means of a clock generator, and also including an analog switch for bypassing the notch filter. A/D converter 156 is shown in Fig 13 Figs 14A and 14B illustrate controller 158, including another 8051-type microcontroller MPU2, which analyzes the ECG signal and generates the trigger pulse.

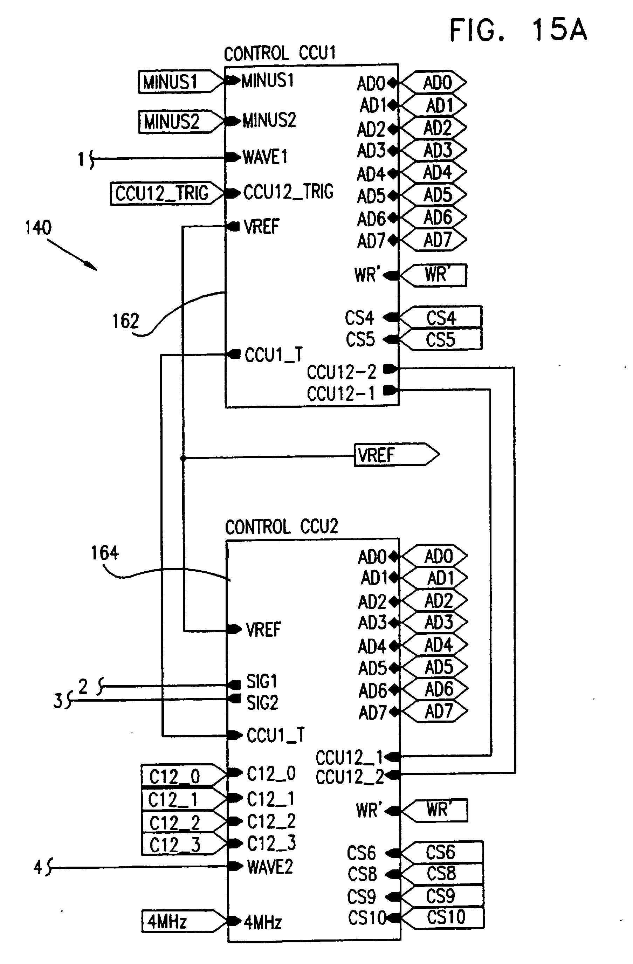

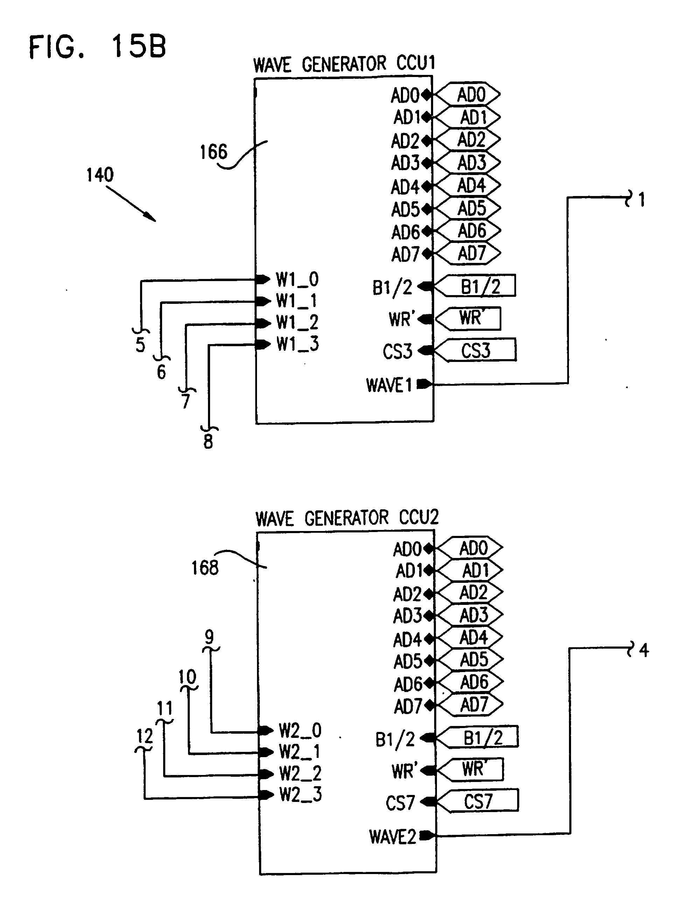

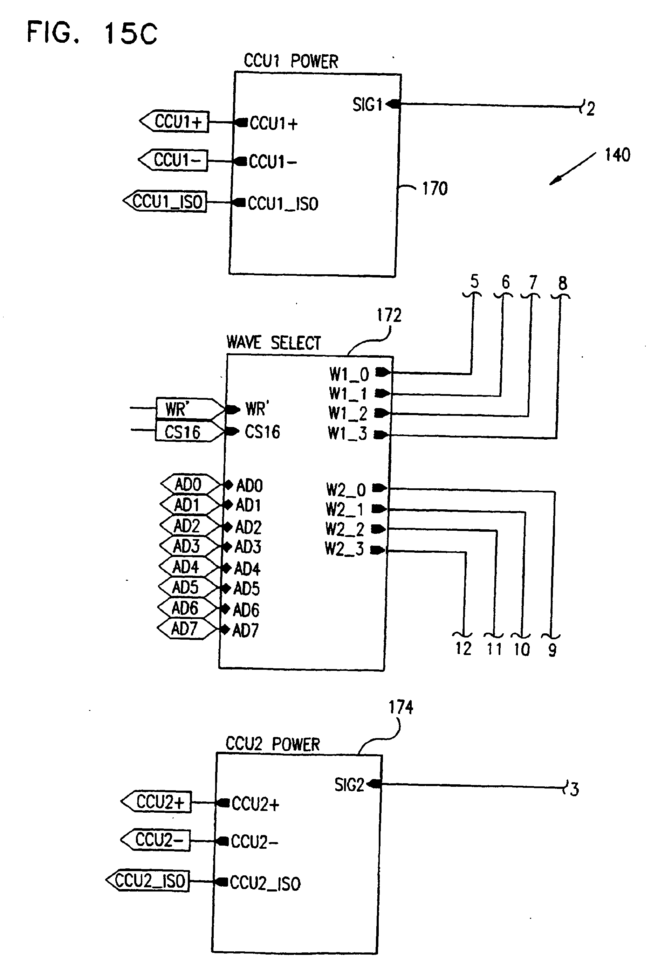

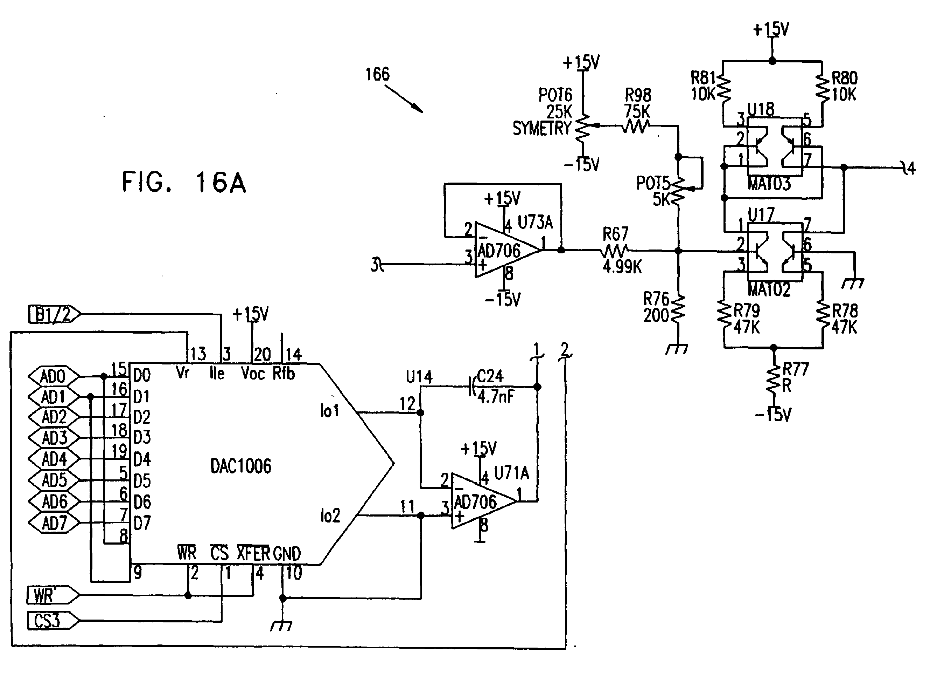

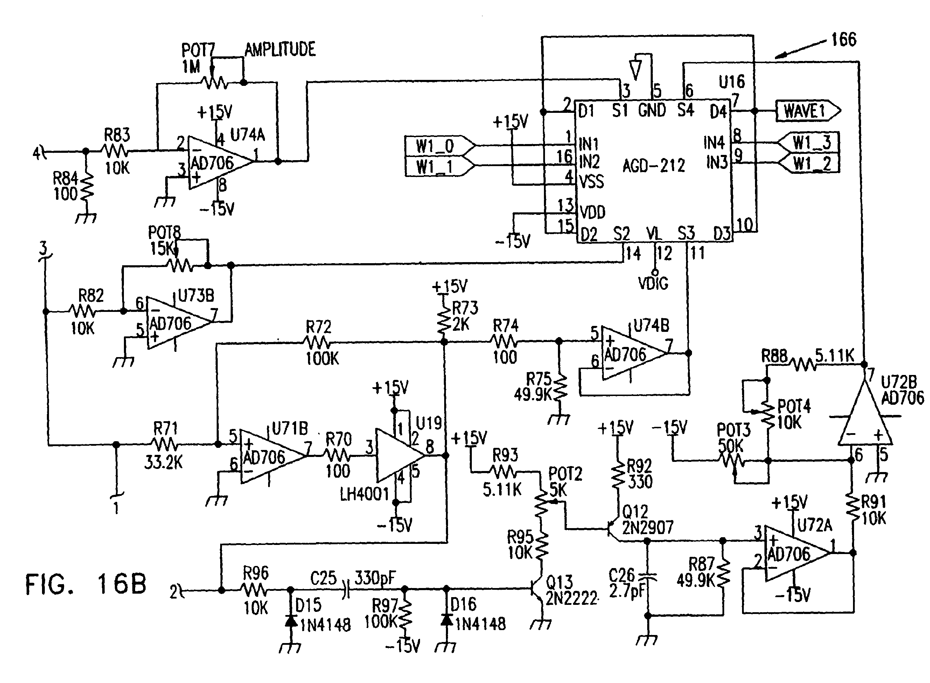

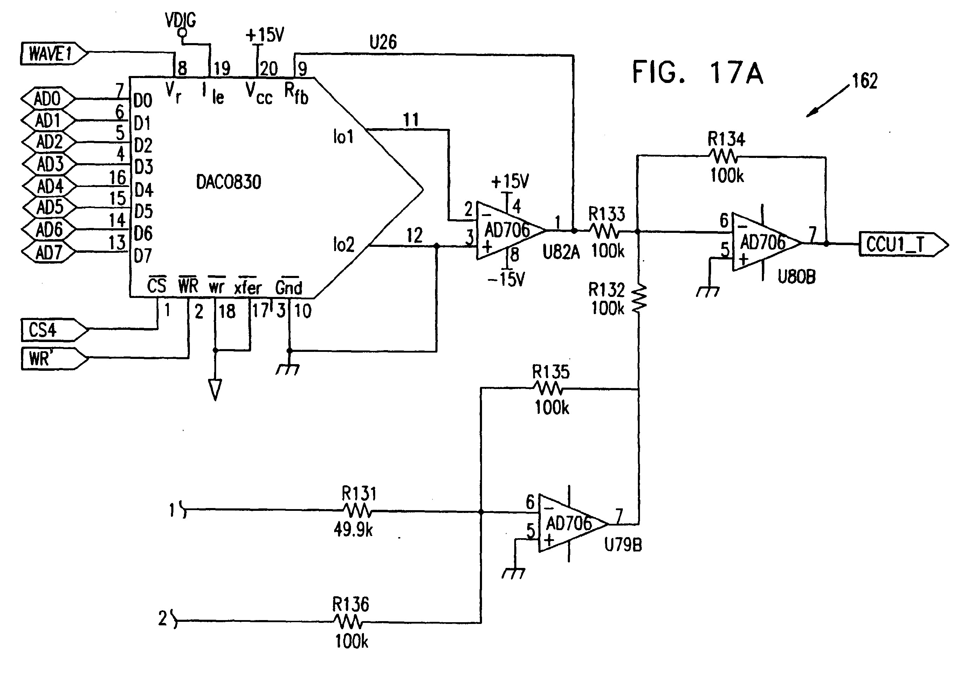

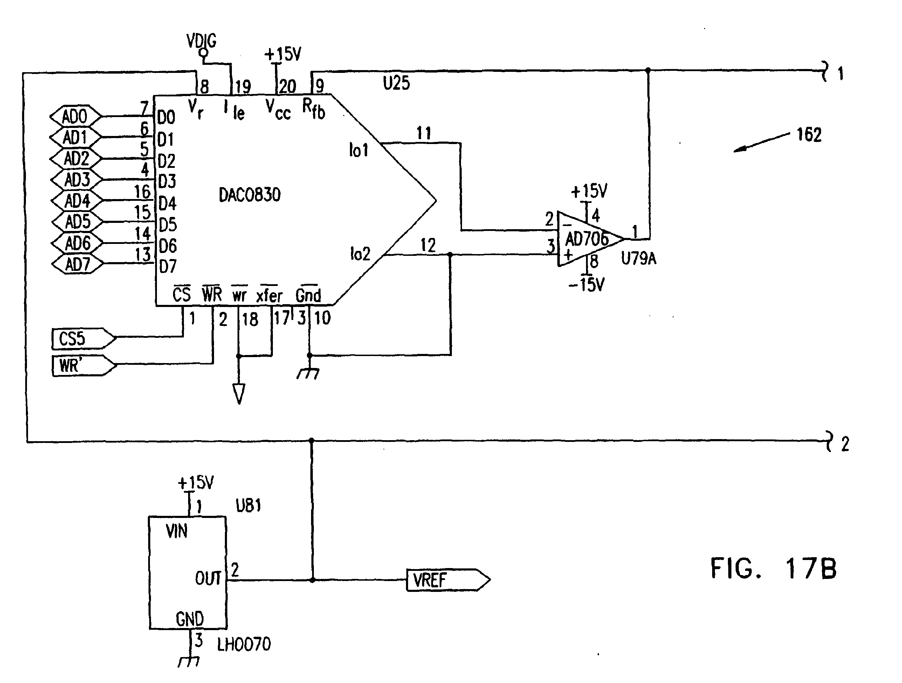

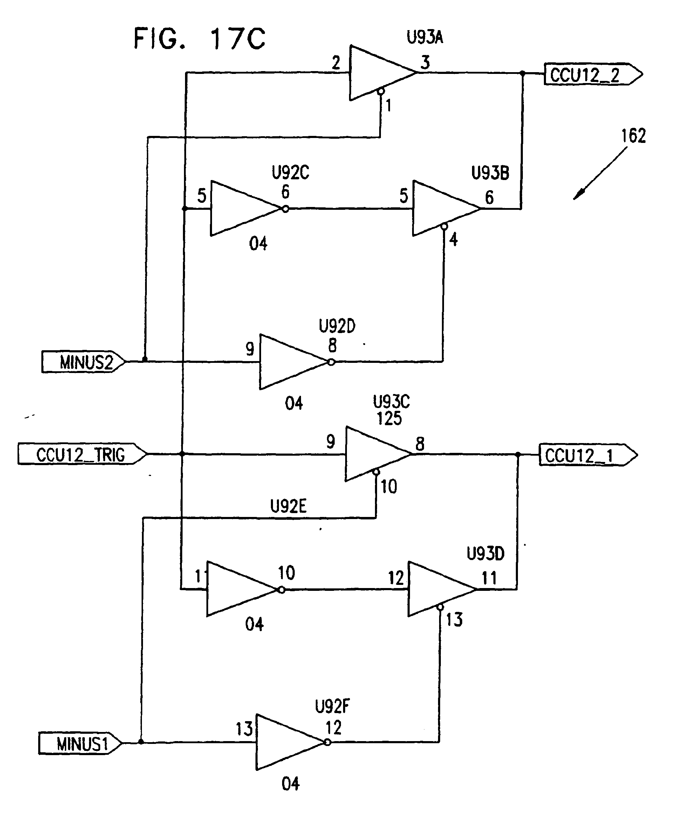

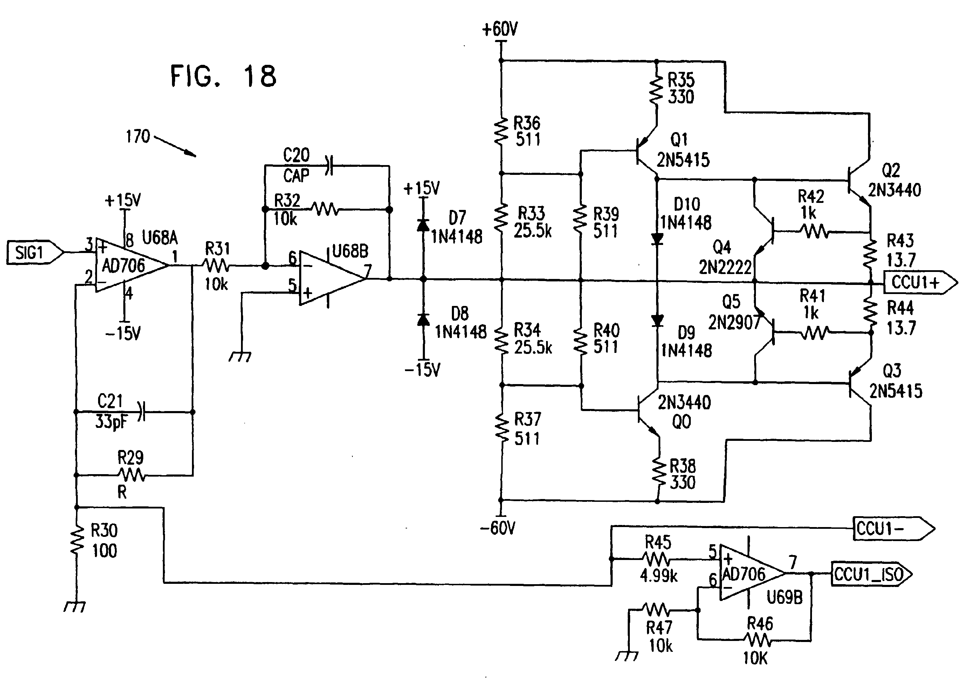

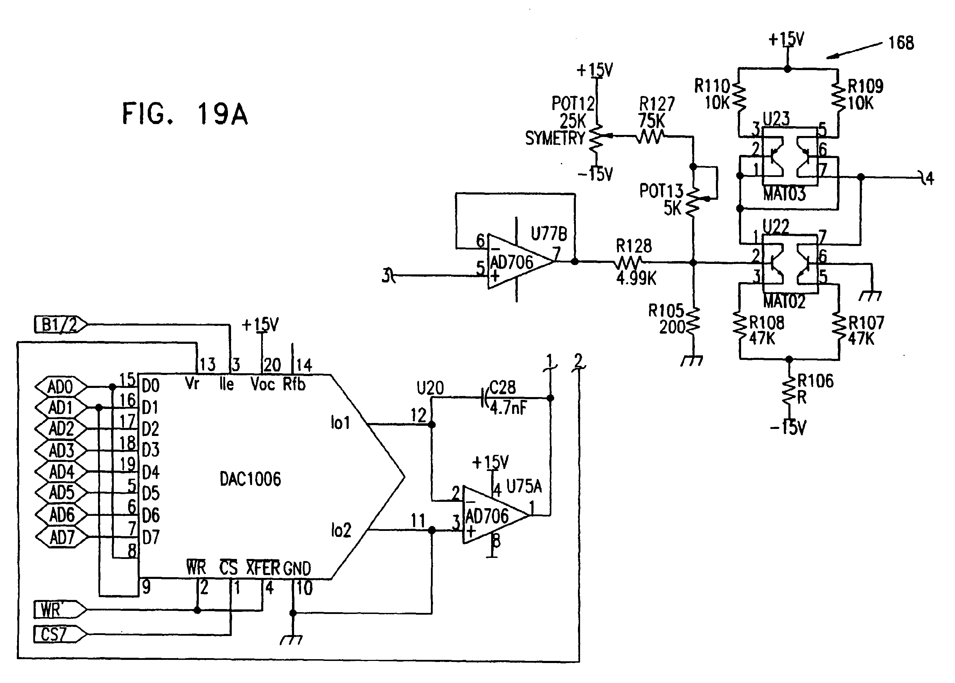

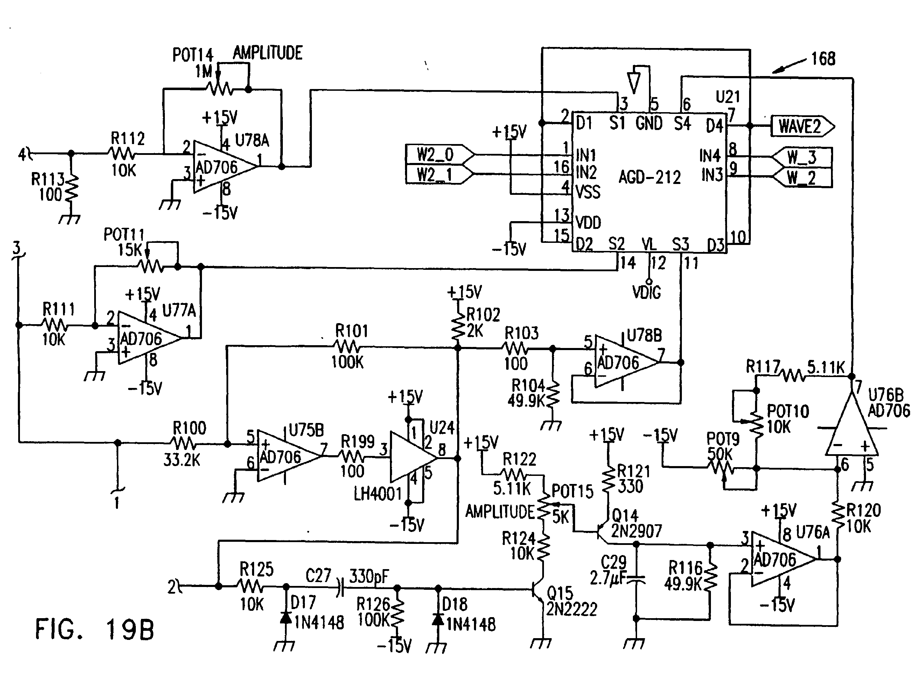

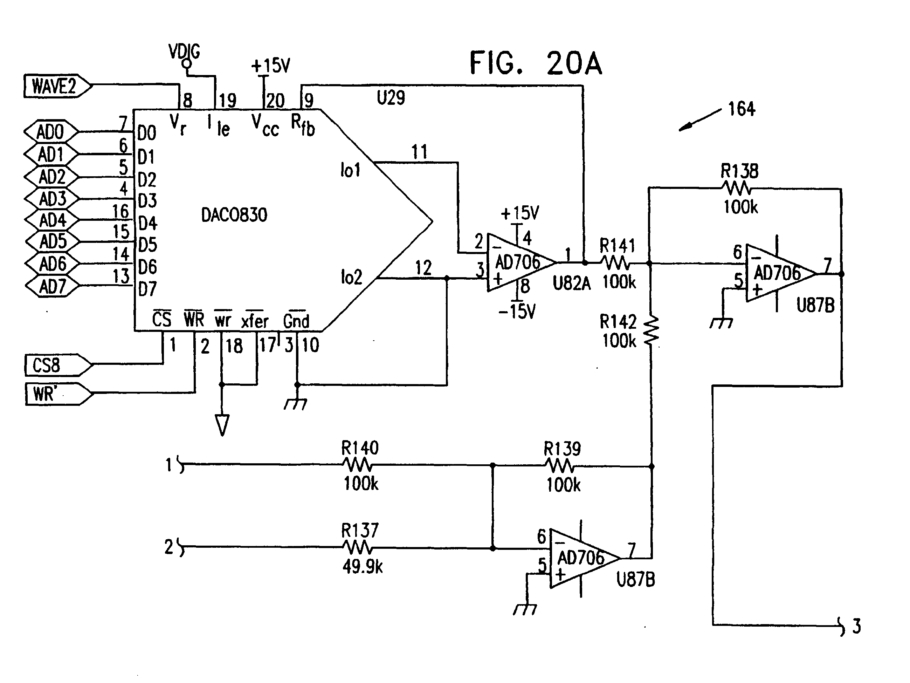

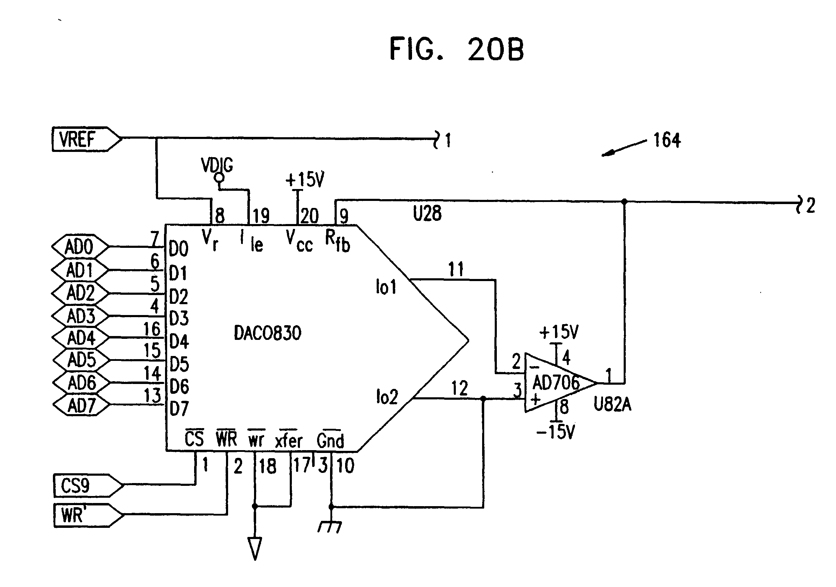

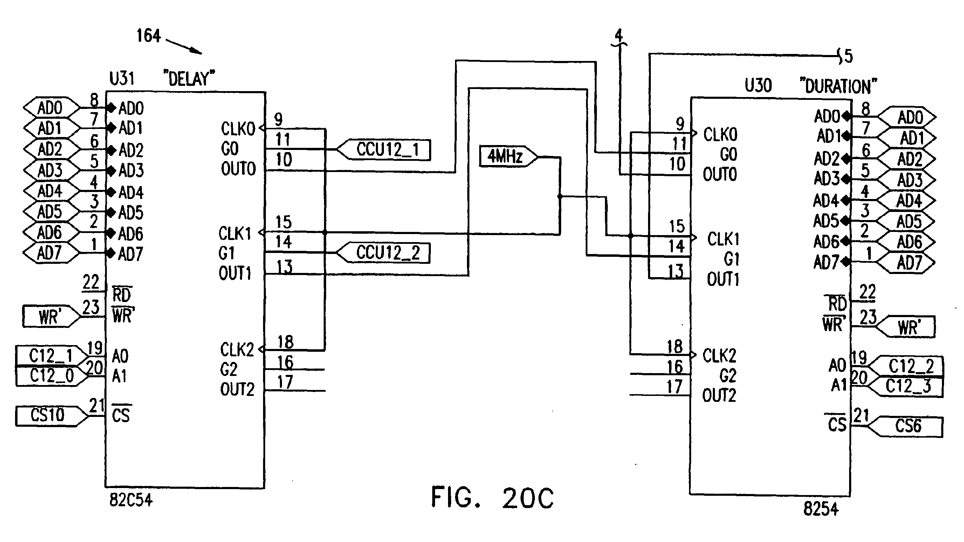

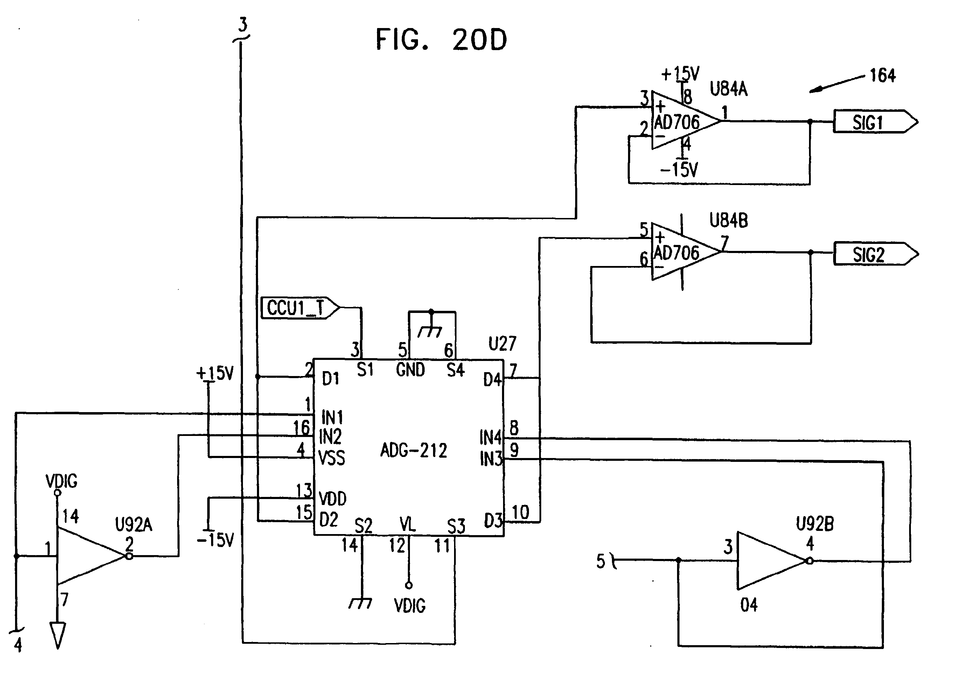

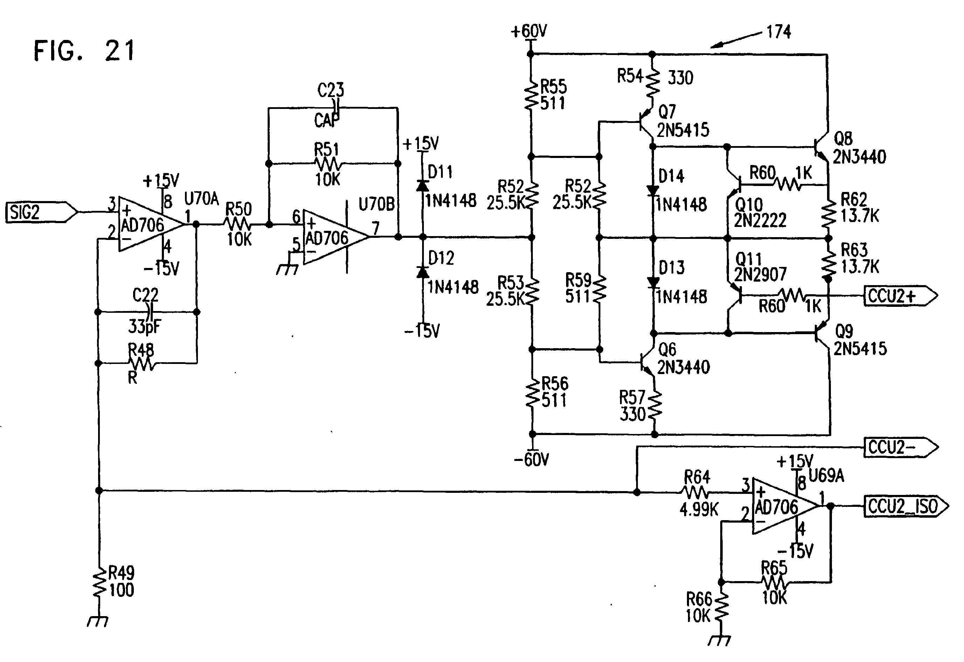

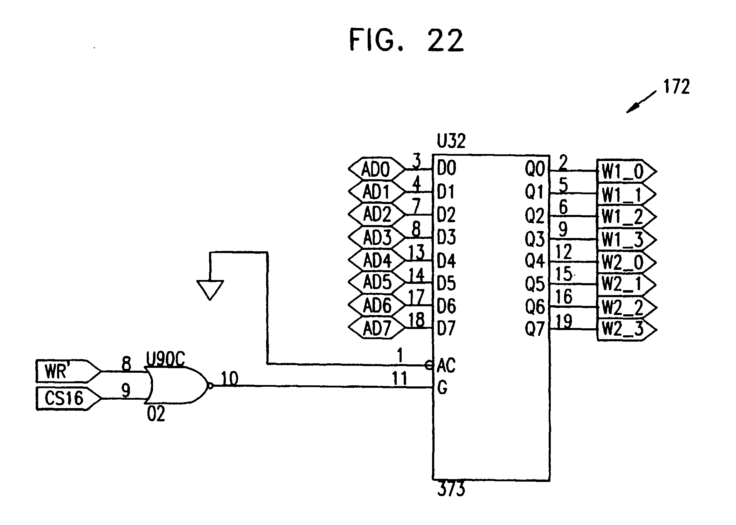

Figs. 15A, 15B and 15C illustrate first CCU section 140, which generates two channels of non-excitatory stimulation pulses. CCU section 140 includes two control units 162 and 164, waveform generators 166 and 168, power units 170 and 174, and a waveform selector 172. Figs. 16A and 16B show details of waveform generator 166, which drives a first one of the two non-excitatory channels, while Figs. 19A and 19B show waveform generator 168, which is substantially similar to generator 166 and drives the second channel Figs. 17A, 17B and 17C illustrate control unit 162, which receives and scales the waveform from generator 166. Fig. 17C shows timing control logic common to both control units 162 and 164. Figs. 20A, 20B, 20C and 20D illustrate control unit 164, wherein Figs. 20A and 20B show waveform scaling circuitry similar to that in Figs. 17A and 17B Figs 20C and 20D include circuitry for controlling the relative delays of the pulses generated by the two stimulation channels. Figs. 18 and 21 show details of power units 174 and 178, respectively, and Fig. 22 illustrates wave selector 176.

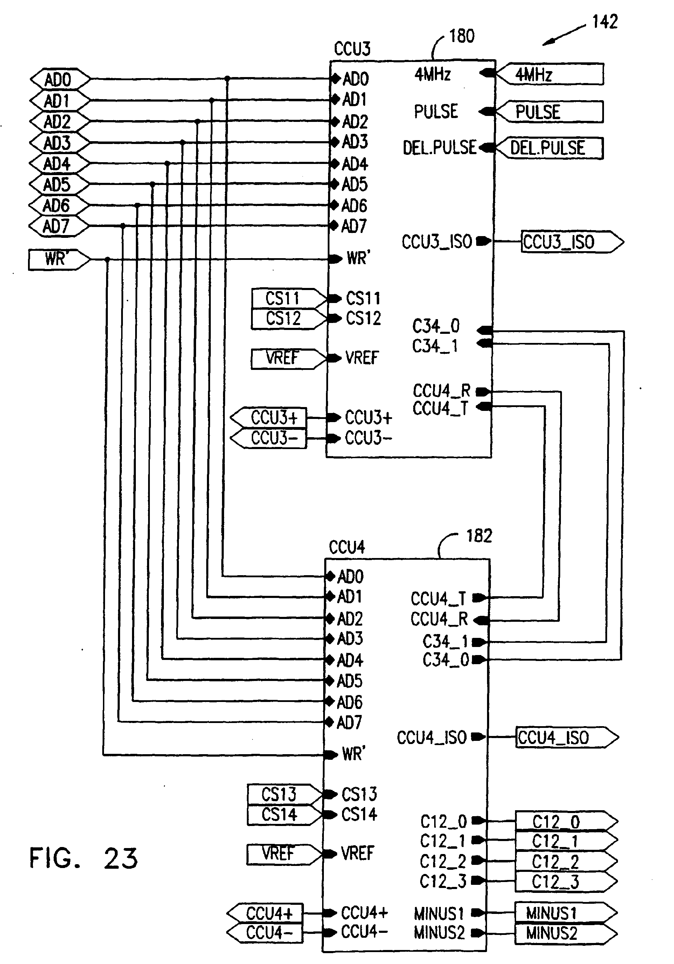

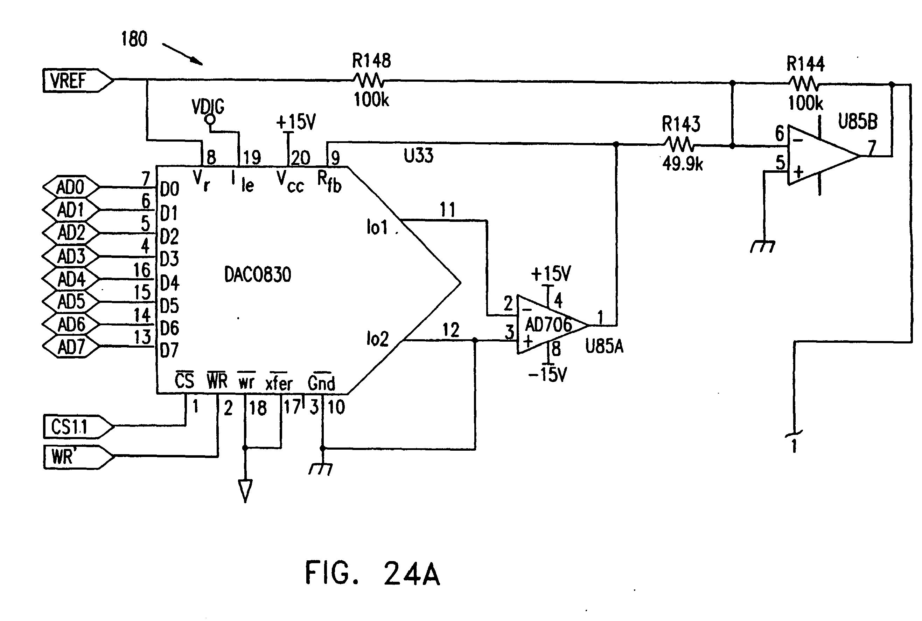

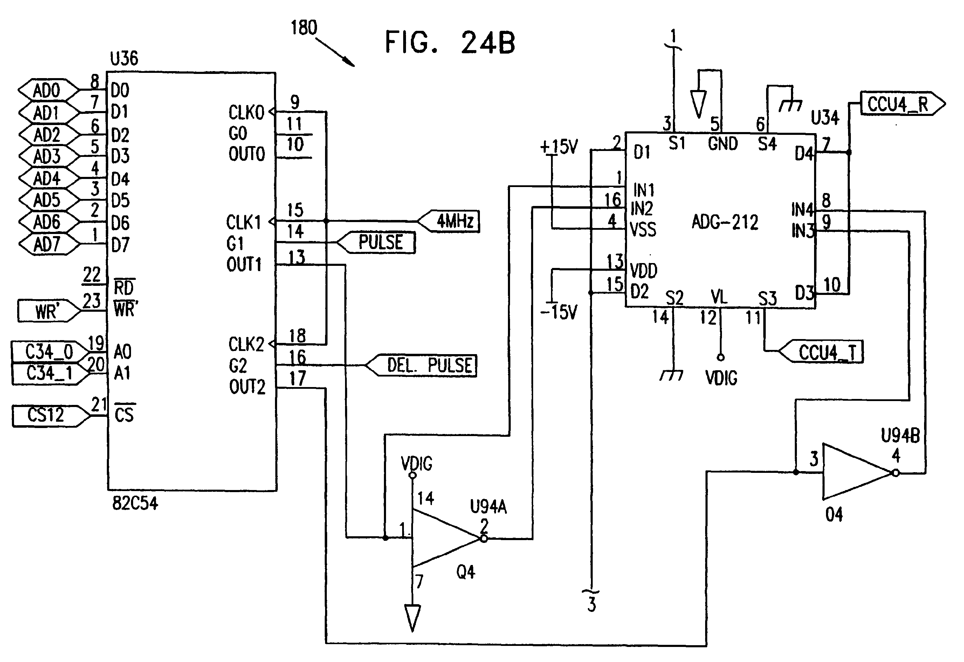

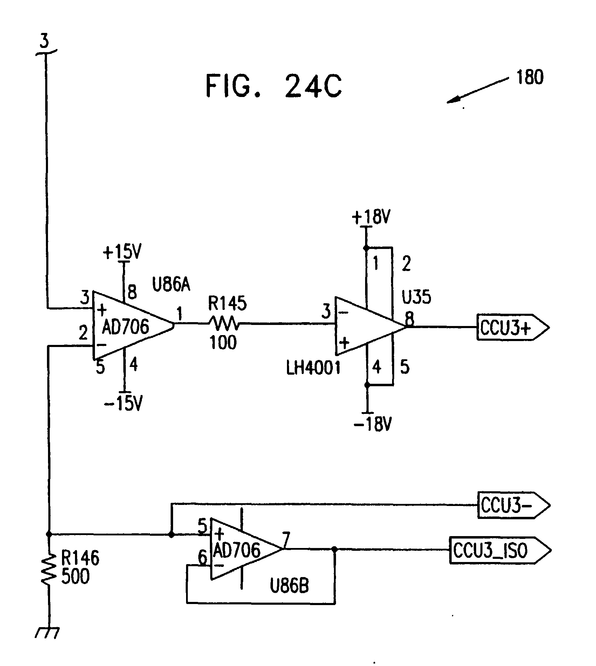

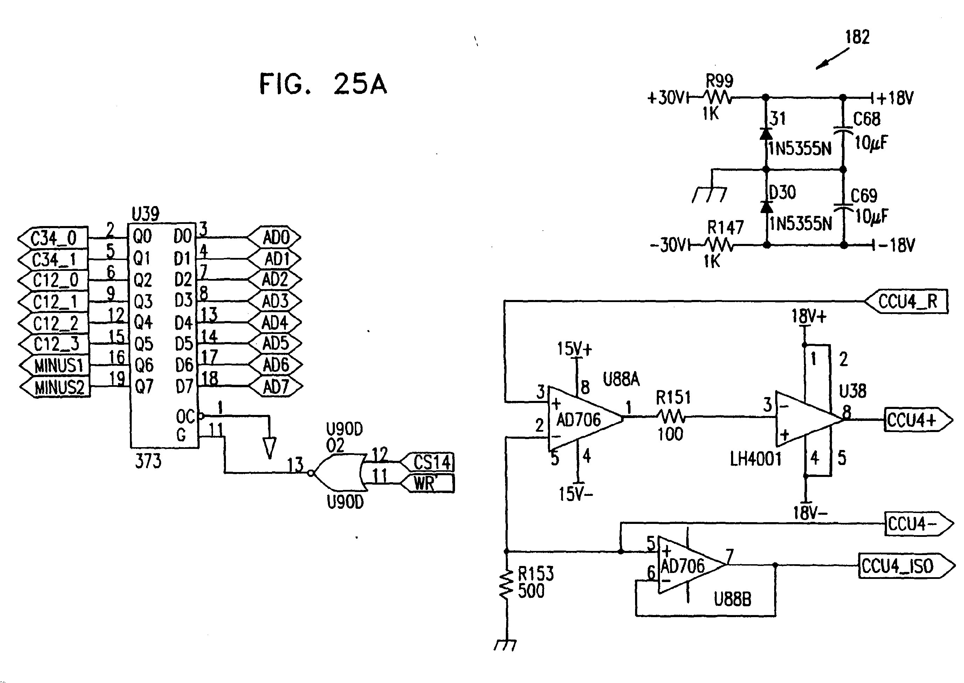

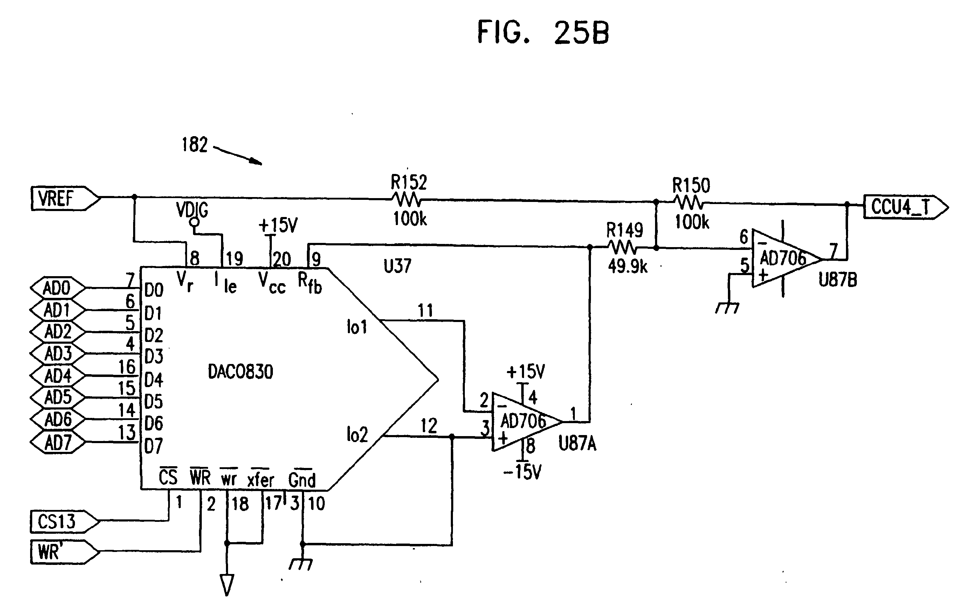

Fig. 23 shows second CCU section 142, including two CCU channels 180 and 182, for generating pacing pulses at predetermined rates and a relative delay therebetween, similar to pacemakers known in the art Figs. 24A, 24B and 24C show details of channel 180. Figs. 25A and 25B show details of channel 182, which is switched by the same switch and counters as channel 180 (shown in Fig. 24B).

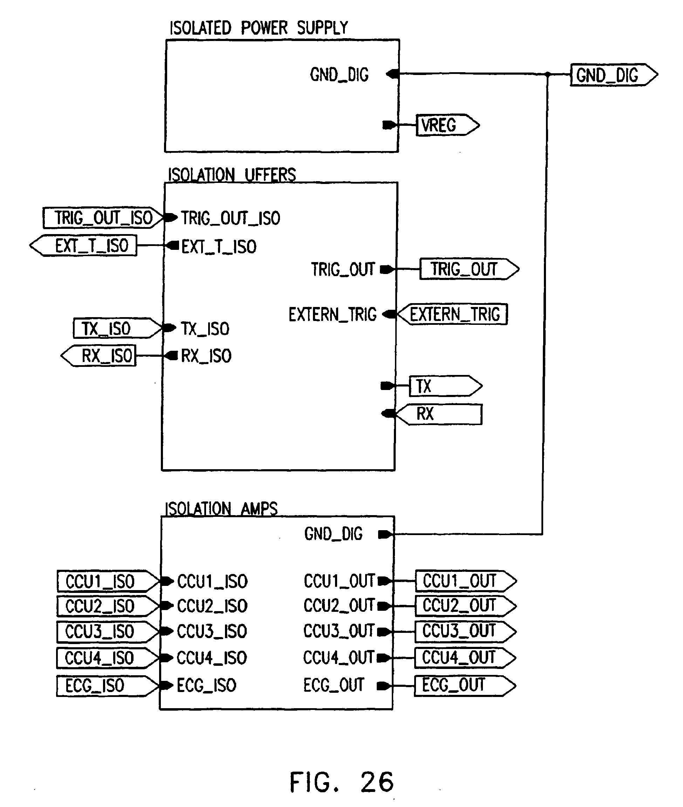

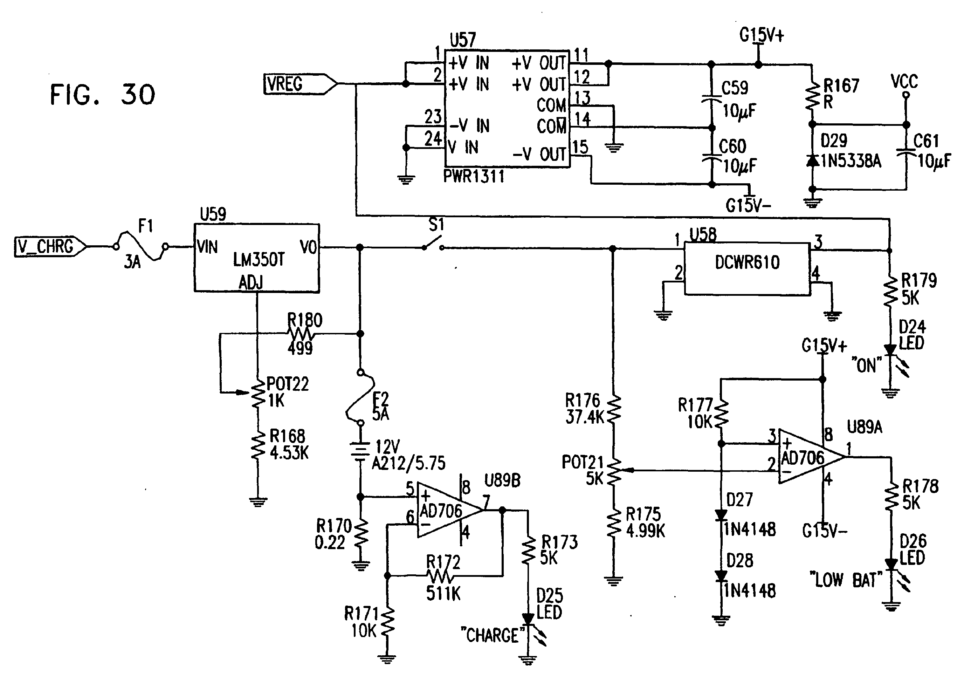

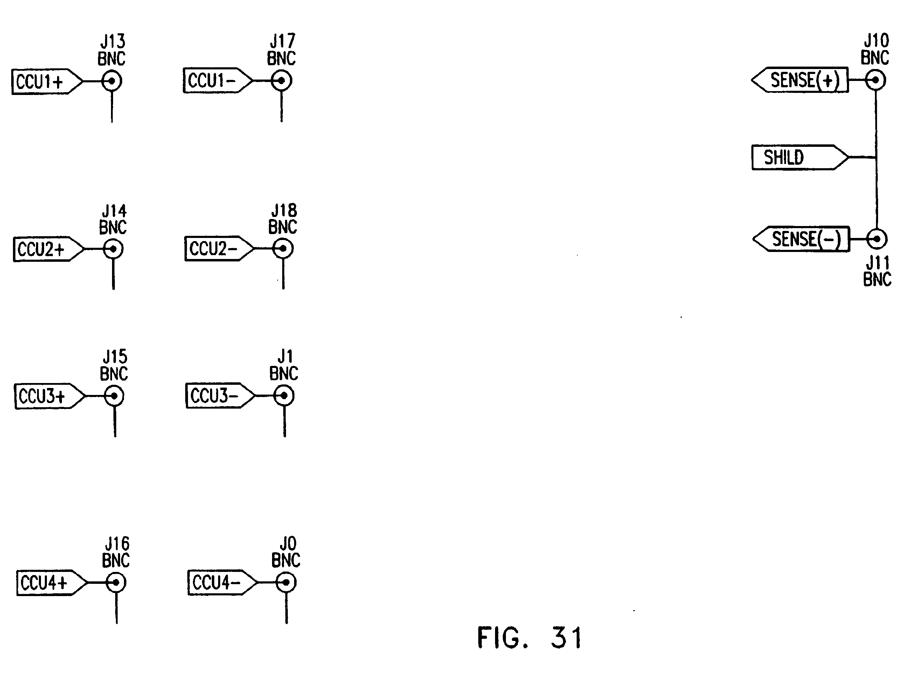

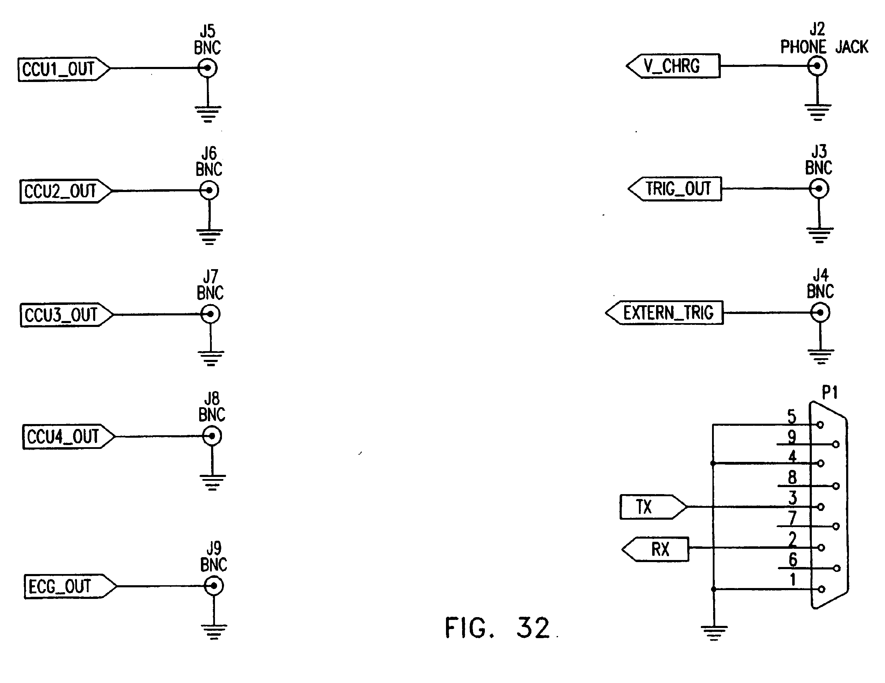

Figs. 26, 27A, 27B, 28, 29A and 29B show details of isolation circuitry, which is used when circuitry 22 is to be run while connected to external power. Fig. 30 illustrates a battery charging circuit. Figs. 31 and 32 show front and rear panel connections, respectively.

Although in some of the preferred embodiments described above, as shown in Fig. 1B, for example, circuitry 22 is shown as being contained within an implantable case 32, the specific implementation of the circuitry exemplified by Figs. 8-32 is better suited to be contained in an external, bedside case, for example, control unit 27, shown in Fig. 1A, in accordance with the best mode of the invention as it is practiced at present It will be understood that the circuitry of Figs. 8-32 can be suitably altered and miniaturized to fit in an implantable case, using methods and electronic devices known in the art, particularly such as are currently used in implantable pacemakers On the other hand, under some circumstances, non-excitatory stimulation and cardiac output regulation may be best accomplished using such an external, bedside case, when the cardiac output must be regulated temporarily, for example, during recovery from infarction or surgery.

It will be appreciated that the preferred embodiments described above are cited by way of example, and the full scope of the invention is limited only by the claims.

## Claims

1. Apparatus for therapeutically modifying cardiac output of the heart of a subject, comprising:
at least one sensor (29) which is adapted to sense signals responsive to cardiac activity;
a stimulation probe (21), comprising at least one stimulation electrode (23, 35) which is configured to apply non-excitatory stimulation pulses (51) to a cardiac muscle segment; and
signal generation circuitry (22), coupled to said at least one sensor (29) and to said stimulation probe (21), which
(i) receives the signals from said at least one sensor
(ii) determines the magnitude and timing of said non- excitatory stimulation pulses (51) responsive to said sensed signals and responsive to said desired change
(iii) generates said non-excitatory stimulation pulses (51) and
(iv) applies said non-excitatory stimulation pulses (51) to said at least one simulation electrode (23, 35),
wherein said magnitude and timing of said non- excitatory stimulation pulses (51) are determined such that
said pulses (51) are applied at a timing and magnitude where they do not generate a propagating action potential, **characterised in that**
said pulses (51) are applied at a timing and magnitude where they produce said desired change of said cardiac output in both an arrhythmic and in a non-arrhythmic heart by changing a stroke volume thereof.

2. Apparatus according to claim 1, **characterised in that** the signal generation circuitry (22) is contained in a unit (20) external to the subject's body.

3. Apparatus according to claim 1, **characterised in that** the signal generation circuitry (22) is contained in an implantable case (32).

4. Apparatus according to any of the preceding claims, **characterised in that** the at least one sensor (29) comprises an intracardiac electrode, and **in that** the signal generation circuitry (22) synchronises the stimulation pulse (51) to electrical activity of the heart.

5. Apparatus according to any of claims 1-3, **characterised in that** the at least one sensor (29) comprises a body surface electrode, and **in that** the signal generation circuitry (22) synchronises the stimulation pulse to an EGG signal.

6. Apparatus according to any of claims 1-3, **characterised in that** the signal generation circuitry (22) identifies an arrhythmia in the signals and controls the stimulation pulses (51) responsive thereto.

7. Apparatus according to any of claims 1-3, **characterised in that** the signal generation circuit (22) detects a QT interval in the signal and controls the stimulation pulses (51) responsive thereto.

8. Apparatus according to any of claims 1-3, **characterised in that** the signal generation circuitry (22) varies at least one parameter of the stimulation pulses (51) from the group of parameters including voltage, current, duration, timing delay, waveform and waveform frequency.

9. Apparatus according to any of claims 1-3, **characterised in that**, after the nonexcitatory stimulation pulse (51), the signal generation circuitry (22) generates another pulse of opposite polarity to the stimulation pulse (51) which is applied to the cardiac muscle segment by the stimulation probe (21).

10. Apparatus according to any of claims 1-3, **characterised in that** the at least one stimulation electrode (23, 35) applies the stimulation pulse (51) to a heart segment having an area of at least 5 mm2.

11. Apparatus according to claim 10, **characterised in that** the at least one stimulation electrode (23, 35) applies the, stimulation pulse (51) to a heart segment having an area of at least 1 cm2.

12. Apparatus according to claim 11, **characterised in that** the at least one stimulation electrode (23, 35) applies the stimulation pulse (51) to a heart segment having an area of at least 4 cm².

13. Apparatus according to claim 10, **characterised in that** the signal generation circuitry (22) varies the area of the heart segment to which the stimulation pulse (51) is applied.

14. Apparatus according to any of claims 1-3, **characterised in that** the stimulation probe (21) comprises a net (40) of electrodes (35).

15. Apparatus according to claim 14, **characterised in that** the electrodes (35) in the net (40) are addressable, such that an extent of the segment to which the stimulation pulse (51) is applied is controlled by addressing selected electrodes (35) in the net (40).

16. Apparatus according to claim 14, **characterised in that** the circuitry (22) applies multiple, different stimulation pulses (51) to different one of the electrodes (35) in the net (40).

17. Apparatus according to claim 16, **characterised in that** the multiple; different stimulation pulses (51) comprises a time sequence of pulses.

18. Apparatus according to claim 4, **characterised in that** the stimulation probe (21) comprises a hybrid electrode (41) including the intracardiac electrode together with at least one of the at least one stimulation electrode (23).

19. Apparatus according to claim 18 **characterised in that** the hybrid electrode (41) comprises a core section including the sensing electrode (42) enclosed within an annular section (32) including the at least one stimulation electrode (23).

20. Apparatus according to claim 19, **characterised in that** the annular section (32) comprises a carbon material.

21. Apparatus according to any of claims 1-3, **characterised in that** the at least one stimulation electrode (23) comprises an elongate electrode, which is inserted into a blood vessel of the heart.

22. Apparatus according to any of claims 1-3, **characterised in that** the at least one stimulation electrode (23) comprises vitreous carbon.

23. Apparatus according to any of claims 1-3, **characterised in that** the at least one stimulation electrode (23) comprises pyrolitic carbon.

24. Apparatus according: to any of claims 1-3, **characterised in that** the at least one sensor (29) comprises a hemodynamic sensor (31), which generates signals responsive to a hemodynamic parameter.

25. Apparatus according to claim 24, **characterised in that** the hemodynamic sensor (31) generates the signals responsive to blood flow.

26. Apparatus according to claim 24, **characterised in that** the hemodynamic sensor (31) generates the signals responsive to blood oxygenation.

27. Apparatus according to claim 24, **characterised in that** the hemodynamic sensor (31) generates the signals responsive to a temperature.

28. Apparatus according to claim 24, **characterised in that** the at least one sensor (29) comprises an electrode, which senses cardiac electrical activity.

29. Apparatus according to any of claims 1-3, and comprising a telemetry device, **characterised by** receiving the signals from at least one of the at least one sensor (29) and by controlling the signal generation circuitry (22) to adjust the non-excitatory stimulation pulse (51).

30. Apparatus according to any of claims 1 or 4-29, **characterised in that** the application of the stimulation pulses (51) engenders an increase in the cardiac output.

31. Apparatus according to any of claims 1 or 4-29, **characterised in that** the application of the stimulation pulses (51) engenders a decrease in the cardiac output.

32. Apparatus according to any of claims 1 or 4-29, **characterised in that** the application of the stimulation pulses (51) increases an efficiency of contraction of the heart.

33. Apparatus according to any of claims 1-32, wherein said non-excitatory pulses are applied at a timing and magnitude at which they do not directly affect heart rate.

34. Apparatus according to any of claims 1-29 or 32, wherein said non-excitatory pulses are applied at a timing and magnitude at which they increase a cardiac output of said heart by increasing a stroke volume of said heart without directly changing a heart rate of said heart.

35. Apparatus according to any of claims 1-29, 32 or 34, wherein said non-excitatory pulses are applied at a timing and magnitude at which they increase cell contractility of cardiac muscle.

36. Apparatus according to any of claims 1-29, 32 or 34, wherein said non-excitatory pulses are applied at a timing and magnitude at which they increase cell contractility of cardiac muscle and wherein said circuitry modifies said increase by changing said determined timing.

## Patentansprüche

1. Vorrichtung zum therapeutischen Modifizieren der Herzleistung des Herzens eines Patienten, umfassend:
wenigstens einen Sensor (29), welcher so ausgelegt ist, dass er Signale als Reaktion auf Herzaktivität erfasst;
eine Reizsonde (21), die wenigstens eine Reizelektrode (23, 35) umfasst, welche so ausgelegt ist, dass sie nicht exzitatorische Reizimpulse (51) an ein Herzmuskelsegment anlegt; und
eine Schaltungsanordnung zur Signalerzeugung (22), welche an den wenigstens einen Sensor (29) und die Reizsonde (21) gekoppelt ist und welche
(i) die Signale von dem wenigstens einen Sensor empfängt,
(ii) die Größe und den Takt der nicht exzitatorischen Reizimpulse (51) als Reaktion auf die erfassten Signale und als Reaktion auf die gewünschte Änderung bestimmt,
(iii) die nicht exzitatorischen Reizimpulse (51) erzeugt, und
(iv) die nicht exzitatorischen Reizimpulse (51) an die wenigstens eine Reizelektrode (23, 35) anlegt,
wobei die Größe und der Takt der nicht exzitatorischen Reizimpulse (51) derart bestimmt werden, dass
die Impulse (51) in einem Takt und einer Größe angelegt werden, in welchen sie kein fortschreitendes Aktionspotenzial erzeugen, **dadurch gekennzeichnet, dass**
die Impulse (51) in einem Takt und einer Größe angelegt werden, in welchen sie die gewünschte Änderung der Herzleistung sowohl bei einem rhythmusgestörten als auch bei einem nicht rhythmusgestörten Herzen durch Ändern eines Schlagvolumens davon erzeugen.

2. Vorrichtung nach Anspruch 1, **dadurch gekennzeichnet, dass** die Schaltungsanordnung zur Signalerzeugung (22) in einer Einheit (20) außerhalb des Körper des Patienten enthalten ist.

3. Vorrichtung nach Anspruch 1, **dadurch gekennzeichnet, dass** die Schaltungsanordnung zur Signalerzeugung (22) in einem implantierbaren Gehäuse (32) enthalten ist.

4. Vorrichtung nach einem der vorhergehenden Ansprüche, **dadurch gekennzeichnet, dass** der wenigstens eine Sensor (29) eine intrakardiale Elektrode umfasst, und **dadurch**, dass die Schaltungsanordnung zur Signalerzeugung (22) den Reizimpuls (51) mit elektrischer Aktivität des Herzens synchronisiert.

5. Vorrichtung nach einem der Ansprüche 1 bis 3, **dadurch gekennzeichnet, dass** der wenigstens eine Sensor (29) eine Körperoberflächenelektrode umfasst, und **dadurch**, dass die Schaltungsanordnung zur Signalerzeugung (22) den Reizimpuls mit einem EGG-Signal synchronisiert.

6. Vorrichtung nach einem der Ansprüche 1 bis 3, **dadurch gekennzeichnet, dass** die Schaltungsanordnung zur Signalerzeugung (22) eine Rhythmusstörung in den Signalen identifiziert und die Reizimpulse (51) als Reaktion darauf steuert.

7. Vorrichtung nach einem der Ansprüche 1 bis 3, **dadurch gekennzeichnet, dass** die Schaltungsanordnung zur Signalerzeugung (22) ein QT-Intervall im Signal erkennt und die Reizimpulse (51) als Reaktion darauf steuert.

8. Vorrichtung nach einem der Ansprüche 1 bis 3, **dadurch gekennzeichnet, dass** die Schaltungsanordnung zur Signalerzeugung (22) wenigstens einen Parameter der Reizimpulse (51) aus der Gruppe von Parametern ändert, die Spannung, Strom, Dauer, Zeitverzögerung, Wellenform und Wellenformfrequenz umfasst.

9. Vorrichtung nach einem der Ansprüche 1 bis 3, **dadurch gekennzeichnet, dass** die Schaltungsanordnung zur Signalerzeugung (22) nach dem nicht exzitatorischen Reizimpuls (51) einen anderen Impuls von entgegengesetzter Polarität zum Reizimpuls (51) erzeugt, welcher durch die Reizsonde (21) an das Herzmuskelsegment angelegt wird.

10. Vorrichtung nach einem der Ansprüche 1 bis 3, **dadurch gekennzeichnet, dass** die wenigstens eine Reizelektrode (23, 35) den Reizimpuls (51) an ein Herzsegment mit einer Fläche von mindestens 5 mm² anlegt.

11. Vorrichtung nach Anspruch 10, **dadurch gekennzeichnet, dass** die wenigstens eine Reizelektrode (23, 35) den Reizimpuls (51) an ein Herzsegment mit einer Fläche von mindestens 1 cm² anlegt.

12. Vorrichtung nach Anspruch 11, **dadurch gekennzeichnet, dass** die wenigstens eine Reizelektrode (23, 35) den Reizimpuls (51) an ein Herzsegment mit einer Fläche von mindestens 4 cm² anlegt.

13. Vorrichtung nach Anspruch 10, **dadurch gekennzeichnet, dass** die Schaltungsanordnung zur Signalerzeugung (22) die Fläche des Herzsegments, an welches der Reizimpuls (51) angelegt wird, ändert.

14. Vorrichtung nach einem der Ansprüche 1 bis 3, **dadurch gekennzeichnet, dass** die Reizsonde (21) ein Netz (40) von Elektroden (35) umfasst.

15. Vorrichtung nach Anspruch 14, **dadurch gekennzeichnet, dass** die Elektroden (35) im Netz (40) ansteuerbar sind, derart dass eine Ausdehnung des Segments, an welches der Reizimpuls (51) angelegt wird, durch Ansteuern von ausgewählten Elektroden (35) im Netz (40) gesteuert wird.

16. Vorrichtung nach Anspruch 14, **dadurch gekennzeichnet, dass** die Schaltungsanordnung (22) mehrere verschiedene Reizimpulse (51) an verschiedene der Elektroden (35) im Netz (40) anlegt.

17. Vorrichtung nach Anspruch 16, **dadurch gekennzeichnet, dass** die mehreren verschiedenen Reizimpulse (51) eine Zeitfolge von Impulsen umfassen.

18. Vorrichtung nach Anspruch 4, **dadurch gekennzeichnet, dass** die Reizsonde (21) eine Hybridelektrode (41) umfasst, die die intrakardiale Elektrode zusammen mit wenigstens einer der wenigstens einen Reizelektrode (23) umfasst.

19. Vorrichtung nach Anspruch 18, **dadurch gekennzeichnet, dass** die Hybridelektrode (41) einen Kernabschnitt umfasst, der die Erfassungselektrode (42) umfasst, die innerhalb eines ringförmigen Abschnitts (32) eingeschlossen ist, der die wenigstens eine Reizelektrode (23) umfasst.

20. Vorrichtung nach Anspruch 19, **dadurch gekennzeichnet, dass** der ringförmige Abschnitt (32) ein Kohlenstoffmaterial umfasst.

21. Vorrichtung nach einem der Ansprüche 1 bis 3, **dadurch gekennzeichnet, dass** die wenigstens eine Reizelektrode (23) eine längliche Elektrode umfasst, welche in ein Blutgefäß des Herzens eingeführt wird.

22. Vorrichtung nach einem der Ansprüche 1 bis 3, **dadurch gekennzeichnet, dass** die wenigstens eine Reizelektrode (23) glasförmigen Kohlenstoff umfasst.

23. Vorrichtung nach einem der Ansprüche 1 bis 3, **dadurch gekennzeichnet, dass** die wenigstens eine Reizelektrode (23) pyrolytischen Kohlenstoff umfasst.

24. Vorrichtung nach einem der Ansprüche 1 bis 3, **dadurch gekennzeichnet, dass** der wenigstens eine Sensor (29) einen hämodynamischen Sensor (31) umfasst, welcher Signale als Reaktion auf einen hämodynamischen Parameter erzeugt.

25. Vorrichtung nach Anspruch 24, **dadurch gekennzeichnet, dass** der hämodynamische Sensor (31) die Signale als Reaktion auf Blutfluss erzeugt.

26. Vorrichtung nach Anspruch 24, **dadurch gekennzeichnet, dass** der hämodynamische Sensor (31) die Signale als Reaktion auf Sauerstoffanreicherung im Blut erzeugt.

27. Vorrichtung nach Anspruch 24, **dadurch gekennzeichnet, dass** der hämodynamische Sensor (31) die Signale als Reaktion auf eine Temperatur erzeugt.

28. Vorrichtung nach Anspruch 24, **dadurch gekennzeichnet, dass** der wenigstens eine Sensor (29) eine Elektrode umfasst, welche elektrische Herzaktivität erfasst.

29. Vorrichtung nach einem der Ansprüche 1 bis 3 und ein Telemetriegerät umfassend, **gekennzeichnet durch** Empfangen der Signale von wenigstens einem des wenigstens einen Sensors (29) und **durch** Steuern der Schaltungsanordnung zur Signalerzeugung (22), um den nicht exzitatorischen Reizimpuls (51) anzupassen.

30. Vorrichtung nach einem der Ansprüche 1 oder 4 bis 29, **dadurch gekennzeichnet, dass** das Anlegen der Reizimpulse (51) eine Steigerung der Herzleistung bewirkt.

31. Vorrichtung nach einem der Ansprüche 1 oder 4 bis 29, **dadurch gekennzeichnet, dass** das Anlegen der Reizimpulse (51) eine Abnahme der Herzleistung bewirkt.

32. Vorrichtung nach einem der Ansprüche 1 oder 4 bis 29, **dadurch gekennzeichnet, dass** das Anlegen der Reizimpulse (51) eine Wirksamkeit der Kontraktion des Herzens erhöht.

33. Vorrichtung nach einem der Ansprüche 1 bis 32, wobei die nicht exzitatorischen Impulse in einem Takt und einer Größe angelegt werden, in welchen sie die Herzfrequenz nicht direkt beeinflussen.

34. Vorrichtung nach einem der Ansprüche 1 bis 29 oder 32, wobei die nicht exzitatorischen Impulse in einem Takt und einer Größe angelegt werden, in welchen sie eine Herzleistung des Herzens durch Erhöhen eines Schlagvolumens des Herzens ohne direkte Änderung einer Herzfrequenz des Herzens steigern.

35. Vorrichtung nach einem der Ansprüche 1 bis 29, 32 oder 34, wobei die nicht exzitatorischen Impulse in einem Takt und einer Größe angelegt werden, in welchen sie die Zellkontraktionsfähigkeit des Herzmuskels erhöhen.

36. Vorrichtung nach einem der Ansprüche 1 bis 29, 32 oder 34, wobei die nicht exzitatorischen Impulse in einem Takt und einer Größe angelegt werden, in welchen sie die Zellkontraktionsfähigkeit des Herzmuskels erhöhen, und wobei die Schaltungsanordnung die Erhöhung durch Ändern des bestimmten Taktes modifiziert.

## Revendications

1. Appareil pour modifier thérapeutiquement le débit cardiaque du coeur d'un patient, comprenant :
au moins un capteur (29) qui est adapté pour détecter des signaux en réponse à l'activité cardiaque ;
une sonde de stimulation (21) comprenant au moins une électrode de stimulation (23, 35) qui est configurée pour appliquer des impulsions de stimulation sans excitation (51) sur un segment de muscle cardiaque ; et
des circuits de génération de signaux (22), couplés audit au moins un capteur (29) et à ladite sonde de stimulation (21), qui :
(i) reçoivent les signaux dudit au moins un capteur,
(ii) déterminent la grandeur et le temps desdites impulsions de stimulation sans excitation (51) en réponse auxdits signaux détectés et en réponse audit changement souhaité,
(iii) génèrent lesdites impulsions de stimulation sans excitation (51), et
(iv) appliquent lesdites impulsions de stimulation sans excitation (51) sur ladite au moins une électrode de stimulation (23, 35),
dans lequel ladite grandeur et ledit temps desdites impulsions de stimulation sans excitation (51) sont déterminés de sorte que :
lesdites impulsions (51) sont appliquées à un temps et une grandeur où elles ne génèrent pas un potentiel d'action de propagation, **caractérisé en ce que :**
lesdites impulsions (51) sont appliquées à un temps et une grandeur où elles produisent ledit changement souhaité dudit débit cardiaque à la fois sur un coeur arythmique et un coeur non arythmique en changeant son débit systolique.

2. Appareil selon la revendication 1, **caractérisé en ce que** les circuits de génération de signaux (22) sont contenus dans une unité (20) externe au corps du patient.

3. Appareil selon la revendication 1, **caractérisé en ce que** les circuits de génération de signaux (22) sont contenus dans un boîtier implantable (32).

4. Appareil selon l'une quelconque des revendications précédentes, **caractérisé en ce que** le au moins un capteur (29) comprend une électrode intracardiaque, et **en ce que** les circuits de génération de signaux (22) synchronisent l'impulsion de stimulation (51) par rapport à l'activité électrique du coeur.

5. Appareil selon l'une quelconque des revendications 1 à 3, **caractérisé en ce que** le au moins un capteur (29) comprend une électrode de surface corporelle, et **en ce que** les circuits de génération de signaux (22) synchronisent l'impulsion de stimulation par rapport à un signal EGG.

6. Appareil selon l'une quelconque des revendications 1 à 3, **caractérisé en ce que** les circuits de génération de signaux (22) identifient une arythmie dans les signaux et contrôlent les impulsions de stimulation (51) en réponse à ceux-ci.

7. Appareil selon l'une quelconque des revendications 1 à 3, **caractérisé en ce que** les circuits de génération de signaux (22) détectent un intervalle QT dans le signal et contrôlent les impulsions de stimulation (51) en réponse à celui-ci.

8. Appareil selon l'une quelconque des revendications 1 à 3, **caractérisé en ce que** les circuits de génération de signaux (22) modifient au moins un paramètre des impulsions de stimulation (51) du groupe de paramètres comprenant la tension, le courant, la durée, le délai, la forme d'onde et la fréquence de forme d'onde.

9. Appareil selon l'une quelconque des revendications 1 à 3, **caractérisé en ce que**, après l'impulsion de stimulation sans excitation (51), les circuits de génération de signaux (22) génèrent une autre impulsion de polarité opposée à l'impulsion de stimulation (51) qui est appliquée sur le segment de muscle cardiaque par la sonde de stimulation (21).

10. Appareil selon l'une quelconque des revendications 1 à 3, **caractérisé en ce que** la au moins une électrode de stimulation (23, 35) applique l'impulsion de stimulation (51) sur un segment du coeur ayant une surface d'au moins 5 mm².

11. Appareil selon la revendication 10, **caractérisé en ce que** la au moins une électrode de stimulation (23, 35) applique l'impulsion de stimulation (51) sur un segment du coeur ayant une surface d'au moins 1 cm².

12. Appareil selon la revendication 11, **caractérisé en ce que** la au moins une électrode de stimulation (23, 35) applique l'impulsion de stimulation (51) sur un segment du coeur ayant une surface d'au moins 4 cm².

13. Appareil selon la revendication 10, **caractérisé en ce que** les circuits de génération de signaux (22) modifient la surface du segment cardiaque sur laquelle l'impulsion de stimulation (51) est appliquée.

14. Appareil selon l'une quelconque des revendications 1 à 3, **caractérisé en ce que** la sonde de stimulation (21) comprend un ensemble (40) d'électrodes (35).

15. Appareil selon la revendication 14, **caractérisé en ce que** les électrodes (35) dans l'ensemble (40) sont utiles, de sorte qu'une étendue du segment sur laquelle l'impulsion de stimulation (51) est appliquée, est contrôlée en traitant les électrodes sélectionnées (35) dans l'ensemble (40).

16. Appareil selon la revendication 14, **caractérisé en ce que** les circuits (22) appliquent plusieurs impulsions de stimulation différentes (51) sur une électrode différente des électrodes (35) dans l'ensemble (40).

17. Appareil selon la revendication 16, **caractérisé en ce que** les multiples impulsions de stimulation différentes (51) comprennent une séquence temporelle d'impulsions.

18. Appareil selon la revendication 4, **caractérisé en ce que** la sonde de stimulation (21) comprend une électrode hybride (41) comprenant l'électrode intracardiaque conjointement à la au moins une électrode de stimulation (23).

19. Appareil selon la revendication 18, **caractérisé en ce que** l'électrode hybride (41) comprend une section de noyau comprenant l'électrode de détection (42) enfermée à l'intérieur d'une section annulaire (32) comprenant la au moins une électrode de stimulation (23).

20. Appareil selon la revendication 19, **caractérisé en ce que** la section annulaire (32) comprend un matériau en carbone.

21. Appareil selon l'une quelconque des revendications 1 à 3, **caractérisé en ce que** la au moins une électrode de stimulation (23) comprend une électrode allongée, qui est insérée dans un vaisseau sanguin du coeur.

22. Appareil selon l'une quelconque des revendications 1 à 3, **caractérisé en ce que** la au moins une électrode de stimulation (23) comprend du carbone vitreux.

23. Appareil selon l'une quelconque des revendications 1 à 3, **caractérisé en ce que** la au moins une électrode de stimulation (23) comprend du carbone pyrolytique.

24. Appareil selon l'une quelconque des revendications 1 à 3, **caractérisé en ce que** le au moins un capteur (29) comprend un capteur hémodynamique (31) qui génère des signaux en réponse à un paramètre hémodynamique.

25. Appareil selon la revendication 24, **caractérisé en ce que** le capteur hémodynamique (31) génère les signaux en réponse à l'écoulement de sang.

26. Appareil selon la revendication 24, **caractérisé en ce que** le capteur hémodynamique (31) génère les signaux en réponse à l'oxygénation du sang.

27. Appareil selon la revendication 24, **caractérisé en ce que** le capteur hémodynamique (31) génère les signaux en réponse à une température.

28. Appareil selon la revendication 24, **caractérisé en ce que** le au moins un capteur (29) comprend une électrode qui détecte l'activité électrique cardiaque.

29. Appareil selon l'une quelconque des revendications 1 à 3, et comprenant un dispositif de télémesure, **caractérisé en ce qu'**il reçoit les signaux provenant du au moins un capteur (29) et **en ce qu'**il contrôle les circuits de génération de signaux (22) afin d'ajuster l'impulsion de stimulation sans excitation (51).

30. Appareil selon l'une quelconque des revendications 1 ou 4 à 29, **caractérisé en ce que** l'application des impulsions de stimulation (51) engendre une augmentation du débit cardiaque.

31. Appareil selon l'une quelconque des revendications 1 ou 4 à 29, **caractérisé en ce que** l'application des impulsions de stimulation (51) engendre une diminution du débit cardiaque.

32. Appareil selon l'une quelconque des revendications 1 ou 4 à 29, **caractérisé en ce que** l'application des impulsions de stimulation (51) augmente une efficacité de contraction du coeur.

33. Appareil selon l'une quelconque des revendications 1 à 32, dans lequel lesdites impulsions sans excitation sont appliquées à un moment et une grandeur auxquels elles n'affectent pas directement la fréquence cardiaque.

34. Appareil selon l'une quelconque des revendications 1 à 29 ou 32, dans lequel lesdites impulsions sans excitation sont appliquées à un moment et une grandeur auxquels elles augmentent un débit cardiaque dudit coeur en augmentant un débit systolique dudit coeur sans changer directement une fréquence cardiaque dudit coeur.

35. Appareil selon l'une quelconque des revendications 1 à 29, 32 ou 34, dans lequel lesdites impulsions sans excitation sont appliquées à un moment et une grandeur auxquels elles augmentent la capacité de contraction cellulaire du muscle cardiaque.

36. Appareil selon l'une quelconque des revendications 1 à 29, 32 ou 34, dans lequel lesdites impulsions sans excitation sont appliquées à un moment et une grandeur auxquels elles augmentent la capacité de contraction cellulaire du muscle cardiaque et dans lequel lesdits circuits modifient ladite augmentation en modifiant ledit temps déterminé.
